(19) Europäisches Patentamt
European Patent Office
Office européen des brevets

(11) Veröffentlichungsnummer: **0 130 934**
**B1**

(12) # EUROPÄISCHE PATENTSCHRIFT

(45) Veröffentlichungstag der Patentschrift: **05.08.87**

(21) Anmeldenummer: **84730074.6**

(22) Anmeldetag: **28.06.84**

(51) Int. Cl.⁴: **C 07 C 103/50,** C 07 C 102/00, C 07 C 149/243, A 61 K 31/195, A 61 K 49/00

(54) **Neue Komplexbildner, Komplexe und Komplexsalze.**

(30) Priorität: **01.07.83 DE 3324235**

(43) Veröffentlichungstag der Anmeldung: **09.01.85 Patentblatt 85/2**

(45) Bekanntmachung des Hinweises auf die Patenterteilung: **05.08.87 Patentblatt 87/32**

(84) Benannte Vertragsstaaten: **AT BE CH DE FR GB IT LI LU NL SE**

(56) Entgegenhaltungen:
**EP - A - 0 071 564**
**CH - A - 482 647**
**DE - A - 2 028 219**
**DE - A - 2 650 966**
**US - A - 3 024 274**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Gries, Heinz, Dr., Helmstedter Strasse 19, D-1000 Berlin 31 (DE)**
Erfinder: **Renneke, Franz-Josef, Dr., Markelstrasse 45, D-1000 Berlin 41 (DE)**
Erfinder: **Weinmann, Hanns-Joachim, Dr., Westhofener Weg 27, D-1000 Berlin 38 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft den in den Patentansprüchen gekennzeichneten Gegenstand.

Aminopolycarbonsäuren, deren Metallkomplexe und Metallkomplexsalze sowie Verfahren zu deren Herstellung sind bekannt:

vgl. z.B. US-Patent 2 407 645, 2 387 735, 3 061 628, 2 130 505 und 3 780 099, DE-OS 2 918 842 und EP 71 564.

Die Anwendung von Komplexbildnern oder Komplexen bzw. deren Salzen in der Medizin ist seit langem bekannt. Als Beispiele seien genannt:

Komplexbildner als Stabilisatoren pharmazeutischer Präparate, Komplexe und deren Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen,

als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotope wie $^{99m}Tc$ für die Szintigraphie sind bekannt.

In der EP 71 564 sind neuerdings paramagnetische Komplexsalze als NMR-Diagnostika vorgeschlagen worden.

Alle bisher bekannten Komplexbildner, Komplexe und deren Salze bereiten bei ihrer klinischen Anwendung Probleme im Hinblick auf die Verträglichkeit, Selektivität der Bindung und Stabilität. Diese Probleme sind umso ausgeprägter, je höher die Komplexbildner und die aus ihnen abgeleiteten Produkte dosiert werden müssen. Beispielsweise schränkt in der Theraphie von Metallvergiftungen die ungenügende Nierenverträglichkeit der heute verfügbaren Komplexbildner sowie deren Neigung für den Organismus essentielle Ionen zu binden, den Gebrauch ein. Die an und für sich nützliche Anwendung schwerer Elemente als Bestandteile von parenteral zu verabreichenden Röntgenkontrastmitteln scheiterte bisher an der ungenügenden Verträglichkeit derartiger Verbindungen. Bei den bisher für die Kernspintomographie vorgeschlagenen oder geprüften paramagnetischen, kontrastverstärkenden Substanzen ist der Abstand zwischen der wirksamen und der im Tierexperiment toxischen Dosis relativ eng.

Es besteht daher für vielfältige Zwecke ein Bedarf an vor allem besser verträglichen, aber auch stabilen, gut löslichen und hinreichend selektiven Komplexbildnern. Die neuen, hier beschriebenen Verbindungen der allgemeinen Formel I entsprechen diesen Anforderungen. Sie werden durch partielle Umwandlung von Carboxylgruppen an sich bekannter Aminopolycarbonsäuren in Mono- oder Polyhydroxyalkylamidgruppen erhalten. Für diesen Prozess kommen alle dem Fachmann bekannten Synthesemöglichkeiten in Betracht. Ein Beispiel hierfür ist die Umsetzung der Anhydride oder Ester der allgemeinen Formeln II bis IV mit Mono- oder Polyhydroxyalkylaminen der allgemeinen Formel

$$HN\genfrac{}{}{0pt}{}{<R^4}{<R^5} ,$$

wobei $R^4$ einen gerad- oder verzweigtkettigen Mono- oder Polyhydroxyalkylrest und $R^5$ ein Wasserstoffatom, einen niederen gerad- oder verzweigtkettigen gegebenenfalls mono- oder polyhdroxylierten Kohlenwasserstoffrest darstellen. Diese Reste enthalten 2 bis 7, vorzugsweise 2 bis 4 Kohlenstofffatome und 1 bis 5, vorzugsweise 2 bis 4 Hydroxylgruppen. Als geeignete Hydroxyalkylreste seien beispielsweise genannt: 2-Hydroxypropyl, 3-Hydroxypropyl, 1-(Hydroxymethyl)-ethyl, 2,3-Dihydroxypropyl, Tris(hydroxymethyl)-methyl, 2,3-Dihydroxy-1-hydroxymethylpropyl, 2,3,4,5,6-Pentahydroxyhexyl und vorzugsweise 2-Hydroxyethyl, 2-Hydroxy-1-(hydroxymethyl)ethyl, 2,3-Dihydroxypropyl und 2,3,4-Trihydroxybutyl. Die Polyhydroxyalkylamine können vorteilhafterweise auch in geschützter Form zur Reaktion eingesetzt werden, z.B. als O-Acylderivate oder als Ketale. Dies gilt besonders dann, wenn diese Derivate bequemer und billiger herstellbar sind als die Polyhydroxyalkylamine selbst. Ein typisches Beispiel ist das 2-Amino-1-(2,2-dimethyl-1,3-dioxolan-4-yl)-ethanol, das Acetonid des 1-Amino-2,3,4-trihydroxybutans, hergestellt nach DE-OS 3 150 917.

Die nachträgliche Entfernung der Schutzgruppen ist problemlos und kann z.B. durch Behandlung mit einem sauren Ionenaustauscher in wässrig-ethanolischer Lösung erfolgen.

Die Herstellung der Säureanhydride der allgemeinen Formel II kann nach bekannten Verfahren erfolgen, z.B. nach der im US-Patent 3 660 388 bzw. im DE-OS 1 695 050 beschriebenen Verfahrensweise mit Acetanhydrid in Pyridin. Neue Anhydride wurden analog dieser Vorschrift hergestellt. Als Beispiel sei die Synthese von

1,8-Bis(2,6-dioxomorpholin)-3,6-diazaoctan-3,6-diessigsäure beschrieben:

4,95 g (0,01 mol) Triethylentetraminhexaessigsäure werden in 7,5 ml Pyridin suspendiert und eine halbe Stunde auf 40°C erwärmt. Es werden 6 ml Essigsäureanhydrid zugegeben und 24 Stunden auf 65°C erwärmt. Die entstandene dunkelbraune Suspension wird filtriert und der Niederschlag mehrfach zunächst mit Essigsäureanhydrid, dann mit Diethylether gewaschen und an der Luft getrocknet. Man erhält 2,51 g (55% der Theorie) eines hellbraunen Pulvers vom Schmelzpunkt 156°C (Aufschäumen).

Analyse:

| | | | | | | | |
|---|---|---|---|---|---|---|---|
| C | 47,16 | H | 5,72 | N | 12,22 | (Ber.) |
| C | 46,76 | H | 5,84 | N | 11,78 | (Gef.) |

In bestimmten Fällen ist es jedoch von besonderem Vorteil die Wasserabspaltung mit Carbodiimiden in einem geeigneten Lösungsmittel schonend vorzunehmen. Dies sei am Beispiel von 4,4'-(trans-1,2-Cyclohexandiyl)-bis(2,6-morpholindion) erläutert.

34,6 g (0,1 mol) trans-1,2-Cyclohexylendinitrilotetraessigsäure werden in 500 ml alsolutem Dimethylformamid unter Erwärmen auf 80°C gelöst und diese Lösung im Eisbad auf unter 5°C gekühlt. Es werden 41,2 g (0,2 mol) Dicyclohexylcarbodiimid, fest, unter Rühren und Kühlen zugegeben. Bei weiterer Kühlung über 12 Stunden scheidet sich die Hauptmenge des entstandenen Dicyclohexylharnstoffs als weisser voluminöser Niederschlag ab. Es wird filtriert und das Filtrat i.V./max 50°C eingeengt. Aus einem Rückstand von ca. 100 ml kristalliert beim erneuten Kühlen über mehrere Stunden der restliche Dicyclohexylharnstoff vollständig aus. Nach dem Filtrieren wird weiter eingeengt und der Rückstand, ein gelbbraunes Öl, in 200 ml abs. Ethylether verrührt. Dabei fällt das Dianhydrid als leicht gelb gefärbtes Pulver aus. Durch mehrfaches Waschen mit abs. Ethylether lassen sich Dimethylformamid-Reste vollständig entfernen. Man erhält 26,6 g (86% der Theorie) eines fast weissen Pulvers vom Schmelzpunkt 178°C (Aufschäumen).

Analyse:

| | | | |
|---|---|---|---|
| C 54,19 | H 5,85 | N 9,03 | (Ber.) |
| C 53,78 | H 6,05 | N 8,97 | (Gef.) |

Die Herstellung der Monoanhydride der Formel IV kann beispielsweise nach dem im J.A.O.C.S. 59 (2) 105 (1982), siehe C.A. 96 164556 u (1982) beschriebenen Verfahren durch partielle Hydrolyse von Bis-anhydriden erfolgen.

Die Umsetzung der Säureanhydride zu den erfindungsgemässen Hydroxyalkylamiden wird in flüssiger Phase durchgeführt. Geeignete Reaktionsmedien sind beispielsweise Wasser, dipolare aprotische Lösungsmittel wie Acetonitril, N-Methylpyrrolidon, Dimethylformamid, Dimethylacetamid und dergleichen oder Gemische derselben. Die Reaktionstemperaturen liegen bei ca. 0°C bis 100°C, wobei Temperaturen von etwa 20°C bis 80°C bevorzugt sind. Die Reaktionszeiten liegen zwischen 0,5 Stunden und 2 Tagen, vorzugsweise zwischen einer Stunde und 36 Stunden.

Die Herstellung der Ester der allgemeinen Formeln III und IV erfolgt in bekanntner Weise, z.B. III und IV nach den in R.A. Guilmette et al., J. Pharm. Sci. 68 194 (1979) und III nach dem im US-Patent 3 497 535 beschriebenen Verfahren.

Die Aminolyse der Ester erfolgt in flüssiger Phase, z.B. in einem geeigneten höhersiedenden Lösungsmittel wie Dimethylformamid, Dimethylacetamid oder Dimethylsulfoxid. Die Reaktionstemperaturen liegen bei etwa 20°C bis 200°C, wobei Temperaturen von 100°C bis 180°C bevorzugt sind. Die Reaktionszeiten liegen zwischen 2 Stunden und 2 Tagen, wobei Reaktionszeiten zwischen 4 und 36 Stunden bevorzugt sind.

Darüber hinaus können alle dem Fachmann bekannten Methoden zur Umwandlung von Carboxylgruppen in Amidgruppen zur Synthese der erfindungsgemässen Komplexbildner, Komplexe und Komplexsalze der allgemeinen Formel I herangezogen werden, so z.B. die Methode nach Krejcarek und Tucker, Biochem. Biophys. Res. Comm. 77, 581 (1977) über gemischte Anhydride.

Die Herstellung der erfindungsgemässen Metallkomplexe erfolgt in der Weise, wie sie in der EP 71 564 offenbart worden ist. Anstelle der Metalloxide können jedoch auch oft vorteilhaft Metallsalze wie Chloride, Acetate und Carbonate zur Chelatisierung verwendet werden. Gewünschtenfalls kann die Komplexierung so durchgeführt werden, dass ohne Isolierung des Metallkomplexes die zur Anwendung kommende wässrige Lösung der gewünschten Konzentration direkt erhalten wird, wobei gegebenenfalls noch vorhandene Carboxylgruppen durch Zugabe stöchiometrischer Mengen anorganischer und/ oder organischer Basen neutralisiert werden. Als anorganische Base werden beispielsweise Natron- und Kalilauge, als organische Base beispielsweise N-Methylglucamin zur Neutralisation verwendet.

Die in Wasser oder physiologischer Salzlösung gelösten oder suspendierten Komplexverbindungen werden — gegebenenfalls in Form ihrer Salze — mit den in der Galenik üblichen Zusätzen in eine für die intravasale oder enterale Applikation geeignete Form überführt, so dass ihre Konzentrationen in einem Bereich von 0,5 mMol/l bis 1 Mol/l liegen. Die erfindungsgemässen Metallkomplexe bzw. ihre Salze mit physiologisch verträglichen anorganischen und/oder organischen Basen sind wertvolle Therapeutika und Diagnostika.

Sie kommen zur Anwendung:

1. Für die NMR- und Ultraschalldiagnostik in Form ihrer Komplexe mit den Ionen der Übergangsmetalle der Ordnungszahlen 21 bis 29, 42 und 44.

2. Für die NMR-, Röntgen- und Ultraschalldiagnostik in Form ihrer Komplexe mit den Ionen der Lanthanidenelemente der Ordnungszahlen 57 bis 70.

3. Für die Röntgen- und Ultraschalldiagnostik in Form ihrer Komplexe mit den Ionen der Elemente der Ordnungszahlen 71 bis 83.

4. Für die Radiodiagnostik in Form ihrer Komplexe mit den Radioisotopen von z.B. Gallium, Germanium, Technetium, Indium, Ytterbium, Gadolinium.

5. Für die Therapie von Metallvergiftungen als Antidots in Form ihrer Komplexe mit den Elementen der Ordnungszahlen 20 und 30.

6. Für die Radiotherapie in Form ihrer Komplexe mit Radioisotopen, wie z.B. $^{67}Cu$.

Die Anwendung kann in Form von wässrigen Injektions- oder Infusionslösungen, Suspensionen oder Emulsionen erfolgen. Für Leberuntersuchungen eignen sich Einschlussverbindungen mit Liposomen. Im Fall der Verwendung von Radioisotope enthaltenden Komplexen kann deren Herstellung nach den in «Radiotracers for Medical Applications», Volume I CRC Press, Boca Rat on, Florida, beschriebenen Methoden vorgenommen werden.

Überraschenderweise weisen die so erhaltenen erfindungsgemässen Metallchelate nach wie vor sehr hohe Bindungskonstanten auf, d.h. sie besitzen eine gute Stabilität. Offenbar können die Sauerstoff-Funktionen der eingeführten Hydroxyalkylamidgruppen als neue Koordinationspunkte für die Komplexierung dienen. Dies führt zu einer günstigen Stabilität der im physiologischen pH-Bereich zur Anwendung kommenden wässrigen Lösungen der Metallkomplexe. Weiterhin sind die neuen, erfindungsgemässen Komblexbildner überraschenderweise ausreichend selektiv für biologisch nicht essentielle Schwermetalle, so dass beispielsweise die $Cu^{++}$-, $Co^{++}$- und $Fe^{++}$-Ionenkonzentrationen im Körper nicht in unvertretbarer Weise gesenkt werden.

Ein weiterer Vorzug der neuen Erfindungen ist die durch die hydroxylierten Amidgruppen bewirkte hervorragende Wasserlöslichkeit sowohl der Komplexbildner als auch der aus ihnen herzustellenden Komplexe.

Die Verträglichkeit der neuen Komplexbildner, Metallkomplexe und Metallkomplexsalze ist denen der bekannten vergleichbaren Verbindungen eindeutig überlegen. So zeigen beispielsweise sowohl der Gadolinium-II-Komplex der $N^6$-Carboxymethyl-$N^3$,$N^9$-bis(2,3-dihydroxypropyl-N-methyl-carbamoylmethyl)-3,6,9-triazaundecandisäure als auch der Gadolinium-III-Komplex der $N^6$-Carboxymethyl-$N^3$,$N^9$-bis-[bis(2-hydroxy-ethyl)-carbamoylmethyl]-3,6,9-triazaundecandisäure den doppelten Wert der $DL_{50}$ des in der EP 71 564 beschriebenen Di-N-methylglucaminsalzes des Gadolinium-III-Komplexes der Diethylentriaminpentaessigsäure nach i.V.-Gabe bei der Maus. Toxische Wirkungen der bisher zur Salzbildung benötigten Ionen wie Natrium, Kalium, Meglumin usw. entfallen völlig oder grösstenteils. Der osmotische Druck der konzentrierten Lösungen von Komplexbildnern und Komplexen ist drastisch reduziert. Auch dadurch wird die Verträglichkeit nach oraler und parenteraler Gabe wesentlich verbessert, da stark hypertone Lösungen Blutgefässe und Gewebe schädigen, Herz und Kreislauf beeinflussen und unerwünschte diuretische Wirkungen entfalten.

Insgesamt ist es damit gelungen, neue Komplexbildner, Metallkomplexe und Metallkomplexsalze zu synthetisieren, die neue Möglichkeiten in der diagnostischen und therapeutischen Medizin erschliessen. Vor allem die Entwicklung neuartiger bildgebender Verfahren in der medizinischen Diagnostik lässt diese Entwicklung als äusserst wünschenswert und notwendig erscheinen.

Die nachfolgenden Ausführungsbeispiele dienen zur näheren Erläuterung des erfindungsgemässen Verfahrens und der Verwendung der Verfahrensprodukte.

*Beispiel 1*

a) $N^6$-Carboxymethyl-$N^3$,$N^9$-bis(2,3,4-trihydroxybutyl-carbamoylmethyl)-3,6,9-triazaundecandisäure.

$C_{22}H_{41}N_5O_{14}$ MG 599,59

17,9 g (50 mmol) 1,5-Bis(2,6-dioxomorpholino)-3-azapentan-3-essigsäure werden in 50 ml Wasser suspendiert und unter Rühren eine Lösung von 16,1 g (100 mmol) 2-Amino-1-(2,2-dimethyl-1,3-dioxolan-4-yl)-ethanol in 50 ml Wasser zugegeben. Das Reaktionsgemisch geht unmittelbar nach Zugabe des Amins vollständig unter geringer Erwärmung in Lösung. Es wird 12 Stunden bei Raumtemperatur gerührt und die gelbe Lösung über Aktivkohle entfärbt. Nach dem Einengen im Vakuum erhält man 28,4 g (95% der Theorie) eines weissen hygroskopischen Pulvers vom Schmelzpunkt 80 bis 83°C.

Analyse:

| | | | |
|---|---|---|---|
| C 44,07 | H 6,89 | N 11,68 | (Ber.) |
| C 44,21 | H 7,04 | N 11,64 | (Gef.) |

In analoger Weise werden erhalten:

b) durch Umsetzung mit 2,3-Dihydroxypropylamin:

$N^6$-Carboxymethyl-$N^3$,$N^9$-bis(2,3-dihydroxypropyl-carbamoylmethyl)-3,6,9-triazaundecandisäure.

$C_{20}H_{37}N_5O_{12}$ MG 539,54

Ein weisses hygroskopisches Pulver vom Schmelzpunkt 66 bis 70°C.

| | | | |
|---|---|---|---|
| C 44,52 | H 6,91 | N 12,98 | (Ber.) |
| C 44,81 | H 7,12 | N 12,99 | (Gef.) |

c) durch Umsetzung mit N-Methyl-2,3-dihydroxypropylamin:

$N^6$-Carboxymethyl-$N^3$,$N^9$-bis(2,3-dihydroxypropyl-N-methyl-carbamoylmethyl)-3,6,9-triazaundecandisäure.

$C_{22}H_{41}N_5O_{12}$ MG 567,60

Ein weisses hygroskopisches Pulver vom Schmelzpunkt 84 bis 87°C.

| | | | |
|---|---|---|---|
| C 46,55 | H 7,28 | N 12,34 | (Ber.) |
| C 46,64 | H 7,34 | N 12,01 | (Gef.) |

d) durch Umsetzung mit 2-Hydroxy-1-hydroxymethyl-ethylamin:

$N^6$-Carboxymethyl-$N^3$,$N^9$-bis[2-hydroxy-1-(hydroxymethyl)-ethyl-carbamoylmethyl]-3,6,9-triazaundecandisäure.

$C_{20}H_{37}N_5O_{12}$ MG 539,54

Ein weisses hygroskopisches Pulver vom Schmelzpunkt 80 bis 84°C.

| | | | |
|---|---|---|---|
| C 44,52 | H 6,91 | N 12,98 | (Ber.) |
| C 44,53 | H 7,06 | N 12,64 | (Gef.) |

e) durch Umsetzung mit N,N-Bis(2-hydroxyethyl)-amin:

$N^6$-Carboxymethyl-$N^3$,$N^9$-bis[bis(2-hydroxyethyl)-carbamoylmethyl]-3,6,9-triazaundecandisäure.

$C_{22}H_{41}N_5O_{12}$ MG 567,60

Ein weisses hygroskopisches Pulver vom Schmelzpunkt 81 bis 85°C.

| | | | |
|---|---|---|---|
| C 46,55 | H 7,28 | N 12,34 | (Ber.) |
| C 46,57 | H 7,02 | N 12,17 | (Gef.) |

f) durch Umsetzung mit N-Methyl-2,3,4,5,6-pentahydroxyhexyl-amin:

$N^6$-Carboxymethyl-$N^3$,$N^9$-bis(2,3,4,5,6-pentahydroxyhexyl-N-methylcarbamoylmethyl)-3,6,9-triazaundecandisäure.

$C_{28}H_{53}N_5O_{18}$ MG 747,76

Ein weisses hygroskopisches Pulver vom Schmelzpunkt 96 bis 104°C.

| | | | |
|---|---|---|---|
| C 44,98 | H 7,14 | N 9,37 | (Ber.) |
| C 44,71 | H 7,25 | N 9,23 | (Gef.) |

g) durch Umsetzung mit 6-Amino-2,2-dimethyl-1,3-dioxepin-5-ol:

$N^6$-Carboxymethyl-$N^3$,$N^9$-bis[2,3-dihydroxy-propyl-1-(hydroxymethyl)-carbamoylmethyl]-3,6,9-triazaundecandisäure.

$C_{22}H_{41}N_5O_{14}$ MG 599,59

Ein weisses hygroskopisches Pulver vom Schmelzpunkt 92 bis 94°C.

C 44,07 H 6,89 N 11,68 (Ber.)
C 44,15 H 7,02 N 11,50 (Gef.)

*Beispiel 2*

a) trans-1,2-Diamino-N,N'-bis(carboxymethyl)-N,N'-bis(2,3-dihydroxy-propyl-carbamoyl)-cyclohexan.

$C_{20}H_{36}N_4O_{10}$ MG 492,526

15,5 g (50 mmol) 4,4'-(trans-1,2-Tetramethylenethylen)-bis-(2,6-morpholindion) werden in 50 ml Dimethylformamid gelöst und mit 9,1 g (100 mmol) 1-Amino-2,3-propandiol in 50 ml Dimethylformamid versetzt. Es wird 6 Stunden auf 60°C erwärmt und die Lösung im Vakuum eingeengt. Der verbleibende ölige Rückstand wird zunächst mehrfach mit Diethylether und Acetonitril ausgerührt. Reste von Acetonitril werden im Vakuum bei 60°C entfernt. Man erhält 23,4 g (95% der Theorie) eines weissen hygroskopischen Pulvers vom Schmelzpunkt 80 bis 83°C.

Analyse:
C 48,77 H 7,37 N 11,38 (Ber.)
C 48,43 H 7,45 N 11,36 (Gef.)

In analoger Weise werden erhalten:

b) durch Umsetzung mit N-Methyl-2,3-dihydroxypropylamin:

trans-1,2-Diamino-N,N'-bis(carboxymethyl)-N,N'-bis(2,3-dihydroxy-propyl-N-methylcarbamoylmethyl)-cyclohexan.

$C_{22}H_{40}N_4O_{10}$ MG 520,580

Ein weisses hygroskopisches Pulver vom Schmelzpunkt 120 bis 123°C.

C 50,76 H 7,75 N 10,76 (Ber.)
C 50,29 H 7,75 N 10,55 (Gef.)

c) durch Umsetzung mit N,N-Bis(2-hydroxyethyl)-amin:

trans-1,2-Diamino-N,N'-bis(carboxymethyl)-N,N'-bis[bis(2-hydroxy-ethyl)-carbamoylmethyl]-cyclohexan.

$C_{22}H_{40}N_4O_{10}$ MG 520,580

Ein weisses hygroskopisches Pulver vom Schmelzpunkt 70 bis 72°C.

C 50,76 H 7,75 N 10,76 (Ber.)
C 50,24 H 8,26 N 10,49 (Gef.)

d) durch Umsetzen mit 2-Hydroxyethylamin:

trans-1,2-Diamino-N,N-bis(carboxymethyl)-N,N'-bis(2-hydroxy-ethyl-carbamoylmethyl)-cyclohexan.

$C_{18}H_{32}N_4O_8$ MG 432,474

Ein weisses hygroskopisches Pulver vom Schmelzpunkt 97 bis 99°C.

C 49,99 H 7,46 N 12,95 (Ber.)
C 49,75 H 7,60 N 12,87 (Gef.)

e) durch Umsetzung mit 2-Hydroxy-1-(hydroxymethyl)-amin:

trans-1,2-Diamino-N,N'-bis(carboxymethyl)-N,N'-bis[2-hydroxy-1-(hydroxymethyl)-ethylcarbamoylmethyl]-cyclohexan.

$C_{20}H_{36}N_4O_{10}$ MG 492,526

Ein weisses hygroskopisches Pulver vom Schmelzpunkt 102 bis 104°C.

C 48,77 H 7,37 N 11,38 (Ber.)
C 48,25 H 7,26 N 11,18 (Gef.)

*Beispiel 3*

a) $N^3$,$N^6$-Bis(2,3-dihydroxy-propyl-N-methyl-carbamoylmethyl)-3,6-diazaoctandisäure.

$C_{18}H_{34}N_4O_{10}$ MG 466,504

Zu einer heissen Lösung von 19,2 g (193 mmol) 1-N-Methylamino-propan-2,3-diol in 200 ml Acetonitril gibt man ein Lösung von 23,2 g (91,5 mmol) 4,4'-Ethylen-bis(2,6-morpholin-dion) in 600 ml Acetonitril. Man erhitzt 16 Stunden zum Rückfluss und engt dann die Lösung im Vakuum zur Trockne ein. Nach Kodestillation mit Wasser wird im Vakuum zum festen Schaum eingeengt. Nach Trocknen im Vakuum erhält man 36,5 g (86% der Theorie) eines weissen Pulvers vom Schmelzpunkt 76 bis 78°C.

Analyse:
C 46,34 H 7,35 N 12,01 (Ber.)
C 45,90 H 7,68 N 11,82 (Gef.)

b) $N^3$,$N^6$-Bis[2-hydroxy-1-(hydroxymethyl)-ethyl-carbamoylmethyl]-3,6-diazaoctandisäure.

$C_{16}H_{30}N_4O_{10}$ MG 438,452

27,33 g (300 mmol) 2-Hydroxy-1-hydroxymethylethylamin werden in 400 ml Wasser gelöst. Man gibt 38,4 g (0,15 mol) 4,4'-Ethylen-bis(2,6-morpholindion) zu, wobei ein Temperaturanstieg auf 40°C erfolgt. Nach Rühren über Nacht wird die Lösung zum festen Schaum im Vakuum eingeengt. Nach Trocknen im Vakuum erhält man 47 g (72% der Theorie) eines weissen hygroskopischen Pulvers vom Schmelzpunkt 64 bis 67°C.

C 43,83 H 6,90 N 12,78 (Ber.)
C 43,68 H 7,25 N 12,66 (Gef.)

In analoger Weise zu Beispiel 3 b) werden erhalten:

c) durch Umsetzung mit 2,3-Dihydroxypropylamin:

$N^3$,$N^6$-Bis(2,3-dihydroxy-propyl-carbamoylmethyl)-3,6-diazaoctandisäure.

$C_{16}H_{30}N_4O_{10}$ MG 438,452

Ein weisses hygroskopisches Pulver vom Schmelzpunkt 50 bis 52°C.

C 43,83 H 6,90 N 12,78 (Ber.)
C 43,60 H 6,95 N 12,66 (Gef.)

d) durch Umsetzung mit N,N-Bis(2-hydroxyethyl)-amin:

$N^3,N^6$-Bis[bis-(2-hydroxyethyl)-carbamoylmethyl]-3,6-diazaoctandisäure.

$C_{18}H_{34}N_4O_{10}$ MG 466,504

Ein weisses hygroskopisches Pulver vom Schmelzpunkt 48 bis 50°C.

| | | | |
|---|---|---|---|
| C 46,34 | H 7,35 | N 12,01 | (Ber.) |
| C 46,10 | H 7,66 | N 11,88 | (Gef.) |

e) durch Umsetzung mit 6-Amino-2,2-dimethyl-1,3-dioxepin-5-ol:

$N^3,N^6$-Bis[2,3-dihydroxypropyl-1-(hydroxymethylcarbamoylmethyl]-3,6-diazaoctandisäure.

$C_{18}H_{34}N_4O_8$ MG 498,504

Ein weisses hygroskopisches Pulver vom Schmelzpunkt 68 bis 70°C.

| | | | |
|---|---|---|---|
| C 43,37 | H 6,87 | N 11,24 | (Ber.) |
| C 43,10 | H 7,00 | N 11,08 | (Gef.) |

f) durch Umsetzung mit 2-Hydroxyethylamin:

$N^3,N^6$-Bis(2-hydroxyethyl-carbamoylmethyl)-3,6-diazaoctandisäure.

$C_{14}H_{26}N_4O_8$ MG 378,39

Ein weisses hygroskopisches Pulver vom Schmelzpunkt 48 bis 52°C.

| | | | |
|---|---|---|---|
| C 44,44 | H 6,93 | N 14,81 | (Ber.) |
| C 44,25 | H 7,10 | N 14,60 | (Gef.) |

*Beispiel 4*

$N^6,N^9$-Bis(carboxymethyl)-$N^3,N^{12}$-bis(2,3-dihydroxy-propyl-N-methyl-carbamoylmethyl)-3,6,9,12-tetraazatetradecandisäure.

$C_{26}H_{48}N_6O_{14}$ MG 668,70

22,92 g (50 mmol) 1,8-Bis(2,6-dioxomorpholino)-3,6-diazaoctan-3,6-diessigsäure werden in 50 ml Wasser suspendiert und mit 10,51 g (100 mmol) N-Methyl-1-aminopropandiol in 50 ml Wasser innerhalb von 10 Minuten versetzt. Es wird 6 Stunden auf 60°C erwärmt und die klare Lösung im Vakuum bei 50°C eingeengt. Der Rückstand wird im Vakuum bei 60°C getrocknet. Man erhält 31,7 g (95% der Theorie) eines weissen hygroskopischen Pulvers vom Schmelzpunkt 86 bis 92°C.

Analyse:

| | | | |
|---|---|---|---|
| C 46,70 | H 7,24 | N 12,57 | (Ber.) |
| C 46,37 | H 7,03 | N 12,41 | (Gef.) |

*Beispiel 5*

$N^3$-Carboxymethyl-$N^6$-[2-hydroxy-1-(hydroxymethyl)-ethylcarbamoylmethyl]-3,6-diazaoctandisäure.

$C_{13}H_{23}N_3O_9$ MG 365,342

27,4 g (0,1 mol) 2-[4-(2,6-Dioxomorpholino)]-ethylamin-N,N-bis(essigsäure) werden in 200 ml absolutem Dimethylformamid suspendiert und mit 9,1 g (0,1 mol) 2-Amino-1,3-propandiol versetzt. Die Lösung wird 6 Stunden auf 40°C erwärmt, filtriert und im Vakuum eingeengt. Der verbleibende ölige Rückstand wird mehrfach mit Diethylether und Acetonitril ausgerührt. Reste von Acetonitril werden im Vakuum bei 60°C entfernt.

Man erhält 33,2 g (91% der Theorie) eines weissen hygroskopischen Pulvers vom Schmelzpunkt 120 bis 124°C.

Analyse:

| | | | |
|---|---|---|---|
| C 42,74 | H 6,35 | N 11,50 | (Ber.) |
| C 42,21 | H 6,72 | N 11,32 | (Gef.) |

*Beispiel 6*

a) Gadolinium-III-Komplex der $N^6$-Carboxymethyl-$N^3,N^9$-bis(2-3)dihydroxypropyl-N-methyl-carbamoylmethyl)-3,6,9-triazaundecandisäure.

$C_{22}H_{38}GdN_5O_{12}$ MG 721,82

56,8 g (0,10 mol) $N^6$-Carboxymethyl-$N^3,N^9$-bis-(2,3-dihydroxypropyl-N-methyl-carbamoylmethyl)-3,6,9-triazaundecandisäure werden in 250 ml Wasser mit 18,1 g (0,05 mol) Gadoliniumoxid, $Gd_2O_3$, drei Stunden auf 95°C erhitzt. Nach Filtration wird die Lösung im Vakuum zur Trockne gebracht, der Rückstand pulverisiert und im Vakuum bei 50°C getrocknet. Man erhält 74,3 g (98% der Theorie) eines weissen Pulvers vom Schmelzpunkt 266 bis 270°C.

Analyse:

| | | | | |
|---|---|---|---|---|
| C 36,61 | H 5,31 | Gd 21,78 | N 9,70 | (Ber). |
| C 36,49 | H 5,42 | Gd 21,49 | N 9,60 | (Gef.) |

In analoger Weise werden erhalten durch Umsetzung von Gadolinium-III-oxid mit den entsprechenden Komblexbildnern:

b) Gadolinium-III-Komplex der $N^6$-Carboxymethyl-$N^3,N^9$-bis(2,3-dihydroxypropyl-carbamoylmethyl)-3,6,9-triazaundecandisäure.

$C_{20}H_{34}GdN_5O_{12}$ MG 693,77

als weisses Pulver vom Schmelzpunkt 270 bis 274°C.

Analyse:

| | | | | |
|---|---|---|---|---|
| C 34,63 | H 4,94 | Gd 22,67 | N 10,09 | (Ber.) |
| C 34,54 | H 5,10 | Gd 22,31 | N 10,01 | (Gef.) |

c) Gadolinium-III-Komplex der $N^6$Carboxymethyl-$N^3,N^9$-bis[2-hydroxy-1-(hydroxymethyl)-ethyl-carbamoylmethyl]-3,6,9-triazaundecandisäure.

$C_{20}H_{34}GdN_5O_{12}$ MG 693,77

als weisses Pulver vom Schmelzpunkt 295 bis 297°C.

Analyse:

| | | | | |
|---|---|---|---|---|
| C 34,63 | H 4,94 | Gd 22,67 | N 10,09 | (Ber.) |
| C 34,31 | H 4,75 | Gd 22,21 | N 9,78 | (Gef.) |

d) Gadolinium-III-Komplex der $N^6$-Carboxymethyl-$N^3,N^9$-bis[bis(2-hydroxyethyl)-carbamoylmethyl]-3,6,9-triazaundecandisäure.

$C_{22}H_{38}GdN_5O_{12}$ MG 721,82

als weisses Pulver vom Schmelzpunkt 199 bis 202°C.

Analyse:

| | | | | | |
|---|---|---|---|---|---|
| C 36,61 | H 5,31 | Gd 21,78 | N 9,70 | (Ber.) |
| C 36,31 | H 5,70 | Gd 21,39 | N 9,44 | (Gef.) |

e) Gadolinium-III-Komplex der $N^6$-Carboxymethyl-$N^3$,$N^9$-bis(2,3,4--trihydroxybutyl-carbamoylmethyl)-3,6,9-triazaundecandisäure.

$C_{22}H_{38}GdN_5O_{14}$ MG 735,82

als weisses Pulver vom Schmelzpunkt 255 bis 257°C.

Analyse:

| | | | | |
|---|---|---|---|---|
| C 35,05 | H 5,08 | Gd 20,86 | N 9,29 | (Ber.) |
| C 34,88 | H 5,05 | Gd 20,71 | N 9,20 | (Gef.) |

f) Gadolinium-III-Komplex der $N^6$-Carboxymethyl-$N^3$,$N^9$-bis[2,3-dihydroxypropyl-1-(hydroxymethyl)-carbamoylmethyl]-3,6,9-triazaundecandisäure.

$C_{22}H_{38}GdN_5O_{14}$ MG 753,82

als weisses Pulver vom Schmelzpunkt 132 bis 135°C.

Analyse:

| | | | | |
|---|---|---|---|---|
| C 35,05 | H 5,08 | Gd 20,86 | N 9,29 | (Ber.) |
| C 35,17 | H 5,25 | Gd 20,61 | N 9,43 | (Gef.) |

g) Gadolinium-III-Komplex der $N^6$-Carboxymethyl-$N^3$,$N^9$-bis(2,3,4,5,6-pentahydroxyhexyl-N-methyl-carbamoylmethyl)-3,6,9-triazaundecandisäure.

$C_{28}H_{50}GdN_5O_{18}$ MG 901,98

als weisses Pulver vom Schmelzpunkt 158 bis 160°C.

Analyse:

| | | | | |
|---|---|---|---|---|
| C 37,29 | H 5,59 | Gd 17,34 | N 7,76 | (Ber.) |
| C 37,40 | H 5,63 | Gd 17,20 | N 7,54 | (Gef.) |

Ferner werden in analoger Weise erhalten durch Umsetzung verschiedener weiterer Metallsalze mit $N^6$-Carboxymethyl-$N^3$,$N^9$-bis(2,3-dihydroxypropyl-N-methyl-carbamoylmethyl)-3,6,9-triazaundecandisäure:

mit Lanthan-III-oxid, $La_2O_3$

h) Lanthan-III-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}LaN_5O_{12}$ MG 703,47

als weisses Pulver vom Schmelzpunkt 109°C.

Analyse:

| | | | | |
|---|---|---|---|---|
| C 37,56 | H 5,44 | La 19,75 | N 9,96 | (Ber.) |
| C 37,40 | H 5,55 | La 19,60 | N 9,90 | (Gef.) |

mit Dysprosium-III-oxid, $Dy_2O_3$

i) Dysprosium-III-Komplex der $N^6$-Carboxymethyl-$N^3$,$N^9$-bis(2,3)-dihydroxypropyl-N-methyl-carbamoyl)-3,6,9-triazaundecandisäure.

$C_{22}H_{38}DyN_5O_{12}$ MG 727,07

als gelbliches Pulver vom Schmelzpunkt 152°C.

Analyse:

| | | | | |
|---|---|---|---|---|
| C 36,34 | H 5,27 | Dy 22,35 | N 9,63 | (Ber.) |
| C 36,27 | H 5,40 | Dy 22,10 | N 9,52 | (Gef.) |

mit Holmium-III-oxid, $Ho_2O_3$

j) Holmium-III-Komplex der $N^6$-Carboxymethyl-$N^3$,$N^9$-bis(2,3-dihydroxypropyl-carbamoylmethyl)-3,6,9-triazaundecandisäure.

$C_{22}H_{38}HoN_5O_{12}$ MG 729,50

als gelbes Pulver vom Schmelzpunkt 160°C.

Analyse:

| | | | | |
|---|---|---|---|---|
| C 36,22 | H 5,25 | Ho 22,61 | N 9,60 | (Ber.) |
| C 36,15 | H 5,40 | Ho 22,40 | N 9,75 | (Gef.) |

mit Terbium-III-carbonat, $Tb_2(CO_3)_3$

k) Terbium-III-Komplex der $N^6$-Carboxymethyl-$N^3$,$N^9$-(2,3-dihydroxypropyl-N-methyl-carbamoylmethyl)-3,6,9-triazaundecandisäure.

$C_{22}H_{38}N_5O_{12}Tb$ MG 723,49

als rosanes Pulver vom Schmelzpunkt 155°C.

Analyse:

| | | | | |
|---|---|---|---|---|
| C 36,52 | H 5,29 | N 9,68 | Tb 21,97 | (Ber.) |
| C 36,36 | H 5,33 | N 9,61 | Tb 21,80 | (Gef.) |

mit Ytterbium-III-oxid, $Yb_2O_3$

l) Ytterbium-III-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}N_5O_{12}Yb$ MG 737,61

als grünliches Pulver vom Schmelzpunkt 138 bis 140°C.

Analyse:

| | | | | |
|---|---|---|---|---|
| C 35,82 | H 5,19 | N 9,49 | Yb 23,46 | (Ber.) |
| C 35,77 | H 5,30 | N 9,25 | Yb 23,20 | (Gef.) |

mit Wismut-III-oxid, $Bi_2O_3$

m) Wismut-III-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}BiN_5O_{12}$ MG 773,55

als weisses Pulver vom Schmelzpunkt 105°C.

Analyse:

| | | | | |
|---|---|---|---|---|
| C 34,16 | H 4,95 | Bi 27,02 | N 9,05 | (Ber.) |
| C 34,25 | H 5,12 | Bi 26,95 | N 8,98 | (Gef.) |

mit frisch gefälltem Chrom-III-hydroxid, $Cr(OH)_3$

n) Chrom-III-Komplex der $N^6$-Carboxymethyl-$N^3$,$N^9$-(2,3-dihydroxypropyl-N-methyl-carbamoylmethyl)-3,6,9-triazaundecandisäure.

$C_{22}H_{38}CrN_5O_{12}$ MG 616,57

als dunkelviolettes Pulver vom Schmelzpunkt 172 bis 176°C.

Analyse:

| | | | | |
|---|---|---|---|---|
| C 42,86 | H 6,21 | Cr 8,43 | N 11,36 | (Ber.) |
| C 42,57 | H 6,49 | Cr 8,35 | N 11,22 | (Gef.) |

mit frisch gefälltem Eisen-III-oxid, $Fe(OH)_3$

o) Eisen-III-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}FeN_5O_{12}$ MG 620,42

als rotbraunes Pulver vom Schmelzpunkt 102 bis 104°C.

Analyse:

| | | | | |
|---|---|---|---|---|
| C 42,59 | H 6,17 | Fe 9,00 | N 11,29 | (Ber.) |
| C 42,31 | H 6,23 | Fe 9,17 | N 11,04 | (Gef.) |

mit Cer-III-carbonat

p) Cer-III-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}CeN_5O_{12}$ MG 704,70

als weisses Pulver vom Schmelzpunkt 113 bis 115°C.

Analyse:

| | | | | |
|---|---|---|---|---|
| C 37,50 | H 5,43 | Ce 19,88 | N 9,94 | (Ber.) |
| C 37,36 | H 5,70 | Ce 19,67 | N 9,88 | (Gef.) |

mit Samarium-III-carbonat

q) Samarium-III-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}SmN_5O_{12}$ MG 714,93

als rötliches Pulver vom Schmelzpunkt 167°C.

Analyse:

| | | | | |
|---|---|---|---|---|
| C 36,96 | H 5,36 | Sm 21,03 | N 9,80 | (Ber.) |
| C 36,90 | H 5,40 | Sm 21,23 | N 9,65 | (Gef.) |

mit Kupfer-II-hydroxidcarbonat und Natriumhydroxid

r) Natriumsalz vom Kupfer-II-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}CuN_5O_{12}Na$ MG 651,12

als blaues Pulver vom Schmelzpunkt 110 bis 115°C.

Analyse:

| | | | | |
|---|---|---|---|---|
| C 40,58 | H 5,88 | Cu 9,76 | N 10,76 | (Ber.) |
| C 40,40 | H 5,92 | Cu 9,90 | N 10,65 | (Gef.) |

Das Komplexsalz kann das Kupfer auch als Radioisotop $^{67}Cu$ enthalten.

mit Indium-III-oxid

s) Indium-III-Komplex der $N^6$Carboxymethyl-$N^3$,$N^9$-bis(2,3-dihydroxy-propyl-N-methyl-carbamoylmethyl)-3,6,9-triazaundecandisäure.

$C_{22}H_{38}InN_5O_{12}$ MG 677,40

als weisses Pulver vom Schmelzpunkt 152°C.

Analyse:

| | | | | |
|---|---|---|---|---|
| C 38,71 | H 5,65 | In 16,95 | N 10,34 | (Ber.) |
| C 38,84 | H 5,82 | In 16,70 | N 10,10 | (Gef.) |

Der Komplex kann das Metall auch als Radioisotope $^{111}Jn$ oder $^{113m}Jn$ enthalten.

mit Gallium-III-oxid

t) Gallium-III-Komplex der $N^6$-Carboxymethyl-$N^3$,$N^9$-bis(2,3-dihydroxypropyl-N-methyl-carbamoylmethyl)-3,6,9-triazaundecandisäure.

$C_{22}H_{38}GaN_5O_{12}$ MG 634,30

als weisses Pulver vom Schmelzpunkt 132 bis 134°C.

Analyse:

| | | | | |
|---|---|---|---|---|
| C 41,66 | H 6,04 | Ga 10,99 | N 11,04 | (Ber.) |
| C 41,72 | H 6,22 | Ga 11,08 | N 11,20 | (Gef.) |

Der Komplex kann das Metall auch als Radioisotop $^{67}Ga$ enthalten.

*Beispiel 7*

a) Mangan-II-Komplex des trans-1,2-Diamino-N,N'-bis(carboxymethyl)-N,N'-bis(2,3-dihydroxypropyl-carbamoylmethyl)-cyclohexans.

$C_{20}H_{34}MnN_4O_{10}$ MG 545,45

19,70 g (40 mmol) trans-1,2-Diamino-N,N'-bis-carboxymethyl)-N,N'-bis(2,3-dihydroxypropyl-carbamoylmethyl)-cyclohexan werden in 100 ml Wasser gelöst und mit 4,63 g (40 mmol) Mangan-II-carbonat versetzt. Die Suspension wird auf 95°C erhitzt. Unter leichtem Aufschäumen ($CO_2$-Entwicklung) entsteht nach ca. 1 Stunde eine klare Lösung, die 6 Stunden bei Raumtemperatur gerührt wird. Die Lösung wird filtriert, im Vakuum bis zur Trockne eingeengt, der Rückstand getrocknet und pulverisiert. Man erhält 18,54 g (85% der Theorie) eines hellrosa gefärbten Pulvers vom Schmelzpunkt 253 bis 255°C.

Analyse:

| | | | | |
|---|---|---|---|---|
| C 44,04 | H 6,29 | Mn 10,07 | N 10,27 | (Ber.) |
| C 44,10 | H 6,40 | Mn 9,85 | N 9,90 | (Gef.) |

In analoger Weise werden erhalten:

b) Mangan-II-Komplex des trans-1,2-Diamino-N,N'-bis(carboxymethyl)-N,N'-bis[bis(2-hydroxyethyl)-carbamoylmethyl]-cyclohexans.

$C_{22}H_{38}MnN_4O_{10}$ MG 573,50

hellrosa gefärbtes Pulver vom Schmelzpunkt 188 bis 190°C.

Analyse:

| | | | | |
|---|---|---|---|---|
| C 46,07 | H 6,68 | N 9,77 | Mn 9,58 | (Ber.) |
| C 45,79 | H 6,69 | N 9,48 | Mn 9,16 | (Gef.) |

c) Mangan-II-Komplex des trans-1,2-Diamino-N,N'-bis(carboxymethyl)-N,N'-bis(2-hydroxyethyl-carbamoylmethyl)-cyclohexans.

$C_{18}H_{30}MnN_4O_{10}$ MG 485,40

hellrosa gefärbtes Pulver vom Schmelzpunkt 255 bis 257°C.

Analyse:

| | | | | |
|---|---|---|---|---|
| C 44,54 | H 6,23 | N 11,54 | Mn 11,32 | (Ber.) |
| C 44,48 | H 6,10 | N 11,41 | Mn 11,27 | (Gef.) |

d) Mangan-II-Komplex des trans-1,2-Diamino-N,N'-bis(carboxymethyl)-N,N'-bis(2,3-dihydroxypropyl-N-methyl-carbamoylmethyl)-cyclohexans.

$C_{22}H_{38}MnN_4O_{10}$ MG 573,50

hellrosa gefärbtes Pulver vom Schmelzpunkt 223 bis 225°C.

Analyse:

| | | | | | |
|---|---|---|---|---|---|
| C 46,07 | H 6,68 | N 9,77 | Mn 9,58 | (Ber.) |
| C 45,72 | H 6,98 | N 9,38 | Mn 9,20 | (Gef.) |

e) Mangan-II-Komplex des trans-1,2-Diamino-N,N'-bis(carboxymethyl)-N,N'-bis[2-hydroxy-1-(hydroxymethyl)-ethyl-carbamoylmethyl]-cyclohexans.

$C_{20}H_{34}MnN_4O_{10}$ MG 545,45

hellrosa gefärbtes Pulver vom Schmelzpunkt 303 bis 305°C.

Analyse:

| | | | | |
|---|---|---|---|---|
| C 44,04 | H 6,29 | N 10,27 | Mn 10,07 | (Ber.) |
| C 43,63 | H 5,98 | N 10,06 | Mn 9,67 | (Gef.) |

f) Mangan-II-Komplex der $N^3$,$N^6$-Bis(2,3-dihydroxypropyl-N-methyl-carbamoylmethyl)-3,6,9-diazaoctandisäure.

$C_{18}H_{32}MnN_4O_{10}$ MG 519,43

als hellrosa gefärbtes Pulver vom Schmelzpunkt 130°C.

Analyse:

| | | | | |
|---|---|---|---|---|
| C 41,62 | H 6,21 | Mn 10,58 | N 10,79 | (Ber.) |
| C 41,52 | H 6,34 | Mn 10,25 | N 10,71 | (Gef.) |

*Beispiel 8*

Herstellung des Gadolinium-III-Komplexes der $N^3$,$N^9$-Bis(carboxymethyl)-$N^3$,$N^{12}$-bis(2,3-dihydroxypropyl-N-methyl-carbamoylmethyl)-3,6,9,12-tetraazatetradecandisäure.

$C_{26}H_{45}GdN_6O_{14}$ MG 822,93

20,06 g (30 mmol) $N^3$,$N^9$-Bis(carboxymethyl)-$N^3$,$N^{12}$-bis(2,3-dihydroxypropyl-N-methyl-carbamoylmethyl)-3,6,9,12-tetraazatetradecandisäure werden in 100 ml Wasser gelöst und mit 5,44 g (15 mmol) Gadolinium-III-oxid versetzt. Die Suspension wird bis zur klaren Lösung auf 95°C erhitzt, filtriert und die Lösung im Vakuum bei 60°C eingeengt. Der Rückstand wird im Vakuum bei 50°C getrocknet und pulverisiert. Man erhält 23,9 g (97% der Theorie) eines weissen Pulvers vom Schmelzpunkt 186 bis 189°C.

Analyse:

| | | | | |
|---|---|---|---|---|
| C 37,95 | H 5,51 | Gd 19,11 | N 10,21 | (Ber.) |
| C 37,81 | H 5,70 | Gd 19,05 | N 10,17 | (Gef.) |

*Beispiel 9*

Lösung des Gadolinium-III-Komplexes der $N^6$-Carboxymethyl-$N^3$,$N^9$-bis(2,3-dihydroxy-N-methyl-propyl-carbamoylmethyl)-3,6,9-triazaundecandisäure.

a) 283,8 g (0,5 mol) $N^6$-Carboxymethyl-$N^3$,$N^9$-bis-(2,3-dihydroxypropyl-N-methyl-carbamoylmethyl)-3,6,9-triazaundecandisäure werden in Wasser p.i. gelöst. Man gibt 90,63 g (0,25 mol) Gadolinium-III-oxid zu und erhitzt 3 Stunden auf 95°C. Nach Zugabe von 1,2 g Tromethamin wird die neutrale Lösung mit Wasser p.i. auf 1000 ml aufgefüllt, in Flaschen abgefüllt und hitzesterilisiert.

b) 360,9 g (0,5 mol) des unter Beispiel 6 a) erhaltenen Gadolinium-III-Komplexes der $N^6$-Carboxymethyl-$N^3$,$N^9$-bis(2,3-dihydroxypropyl-N-methyl-carbamoylmethyl)-3,6,9-triazaundecandisäure werden in 900 ml Wasser p.i. unter Erwärmen gelöst. Man gibt 1,2 g Tromethamin zu und filtriert die Lösung pyrogenfrei. Dann wird die neutrale Lösung mit Wasser p.i. auf 1000 ml aufgefüllt, in Flaschen abgefüllt und hitzesterilisiert.

c) 178,66 g (0,5 mol) 1,5-Bis(2,6-dioxomorpholino)-3-azapentan-3-essigsäure werden in 300 ml Wasser p.i. suspendiert und mit 105,14 g (1,0 mol) N-Methyl-2,3-dihydroxypropylamin in 100 ml Wasser p.i. versetzt. Es wird 5 Stunden auf 60°C erwärmt, filtriert und die klare Lösung mit 90,62 g (0,25 mol) Gadolinium-III-oxid versetzt. Es wird 6 Stunden auf 95°C erwärmt, filtriert und 1,2 g Tromethamin zugegeben. Man füllt mit Wasser p.i. auf 1000 ml auf, füllt in Flaschen ab und hitzesterilisiert.

Mittel für die NMR-, die Ultraschall- und Röntgendiagnostik.

*Beispiel 10*

Lösung des Mangan-II-Komplexes des trans-1,2-Diamino-N,N'-bis(carboxymethyl)-N,N'-bis[2-hydroxy-1-(hydroxymethyl)-ethyl-carbamoylmethyl]-cyclohexans.

a) 197,01 g (0,4 mol) trans-1,2-Diamino-N,N'-bis(carboxymethyl)-N,N'-bis[2-hydroxy-1(hydroxymethyl)-ethyl-carbamoylmethyl]-cyclohexan werden in 800 ml Wasser p.i. gelöst. Man gibt 45,98 g (0,4 mol) Mangan-II-carbonat portionsweise unter Stickstoff zu. Es wird 3 Stunden bei 95°C gehalten, 1 g Tromethamin zugegeben, die neutrale Lösung steril filtriert und in Flaschen abgefüllt.

b) 218,18 g (0,4 mol) des unter Beispiel 7 e) erhaltenen Mangan-II-Komplexes des trans-1,2-Diamino-N,N'-bis(carboxymethyl)-N,N'-bis[2-hydroxy-1-(hydroxymethyl)-ethyl-carbamoylmethyl]-cyclohexans werden in 900 ml Wasser p.i. gelöst, Man gibt 1 g Tromethamin zu und füllt die neutrale Lösung mit Wasser p.i. auf 1000 ml auf. Die Lösung wird steril filtriert und in Flaschen abgefüllt.

Mittel für die NMR- und Ultraschalldiagnostik.

*Beispiel 11*

Lösung des Natriumsalzes des Calciumkomplexes der $N^6$-Carboxymethyl-$N^3$,$N^9$-bis(2,3-dihydroxypropyl-carbamoylmethyl)-3,6,9-triazaundecandisäure.

269,8 g (0,5 mol) $N^6$-Carboxymethyl-$N^3$,$N^9$-bis-(2,3-dihydroxypropyl-carbamoylmethyl(-3,6,9-triazaundecandisäure werden in 500 ml Wasser p.i. mit 50,04 g (0,5 mol) Calciumcarbonat 5 Stunden zum Rückfluss erhitzt. Man kühlt ab und bringt durch tropfenweise Zugabe von 2n-Natronlauge auf pH 7. Die Lösung wird pyrogenfrei filtriert und für Injektions- oder Infusionszwecke abgefüllt und hitzesterilisiert.

Verwendung als Antidot (Mittel gegen Metallvergiftung).

*Beispiel 12*

$^{99m}$Technetium-Kit der $N^6$-Carboxymethyl-$N^3$,$N^9$-bis(2,3-dihydroxypropyl-carbamoylmethyl)-3,6,9-triazaundecandisäure.

Eine Flasche mit 16,5 mq Trockensubstanz, enthaltend

13,53 mq $N^6$-Carboxymethyl-$N^3$,$N^9$-bis(2,3-dihydroxypropyl-carbamoylmethyl)-3,6,9-triazaundecandisäure
0,17 mg Zinn-II-fluorid
2,8 mg Natriumchlorid

für die $^{99m}$Tc-Markierung zur Szintigraphie.

*Beispiel 13*

Lösung des N-Methylglucaminsalzes des Zink-II-Komplexes der $N^6$-Carboxymethyl-$N^3$,$N^9$-bis(2,3-dihydroxypropyl-N-methyl-carbamoylmethyl)-3,6,9-triazaundecandisäure.

170,28 g (0,30 mol) $N^6$-Carboxymethyl-$N^3$,$N^9$-bis-(2,3-dihydroxypropyl-N-methyl-carbamoylmethyl)-3,6,9-triazaundecandisäure werden in 500 ml Wasser p.i. mit 37,6 g (0,30 mol) Zink-II-carbonat 5 Stunden zum Rückfluss erhitzt. Man setzt portionsweise 56,6 g (0,30 mol) N-Methylglucamin zu, füllt mit Wasser p.i. auf 1000 ml auf, filtriert die Lösung in Flaschen oder Ampullen und unterwirft sie der Hitzesterilisation.

Verwendung als Antidot.

*Beispiel 14*

Lösung des N-Methylglucaminsalzes des Wismut-III-Komplexes der $N^6$,$N^9$-Bis(carboxymethyl)-$N^3$,$N^{12}$-bis-(2,3-dihydroxypropyl-N-methyl-carbamoylmethyl)-3,6,9-tetraazatetradecandisäure.

334,35 g (0,5 mol) $N^6$,$N^9$-Bis(carboxymethyl)-$N^3$,$N^{12}$-bis(2,3-dihydroxypropyl-N-methyl-carbamoylmethyl)-3,6,9,12-tetraazatetradecandisäure werden in 700 ml Wasser p.i. mit 116,5 g (0,25 mol) Wismut-III-oxid 5 Stunden zum Rückfluss erhitzt. Man setzt portionsweise 97,7 g (0,5 mol) N-Methylglucamin zu, füllt mit Wasser p.i. auf 1000 ml auf, filtriert die Lösung in Flaschen und unterwirft sie der Hitzesterilisation.

Mittel für die Röntgendiagnostik.

*Beispiel 15*

Gadolinium-III-Komplex der $N^3$-Carboxymethyl-$N^6$-[2-hydroxy-1-(hydroxymethyl)-ethyl-carbamoylmethyl]-3,6-diazaoctandisäure.

$C_{13}H_{20}GdN_3O_9$ MG 519,567

36,5 g (0,1 mol) $N^3$-Carboxymethyl-$N^6$-[2-hydroxy-1-(hydroxymethyl)-ethyl-carbamoylmethyl]-3,6-diazaoctandisäure werden in 100 ml Wasser gelöst und mit 18,1 g (0,05 mol) Gadolinium-III-oxid versetzt. Die Suspension wird 5 Stunden auf 95°C erhitzt. Die Lösung wird filtriert, im Vakuum eingeengt, der Rückstand getrocknet und pulverisiert.

Man erhält 50,6 g (97% der Theorie) eines weissen Pulvers vom Schmelzpunkt 246 bis 248°C.

Analyse:

| | | | | | |
|---|---|---|---|---|---|
| C 30,05 | H 3,88 | Gd 30,27 | N 8,09 | (Ber.) |
| C 30,19 | H 3,70 | Gd 30,10 | N 7,85 | (Gef.) |

*Beispiel 16*

Herstellung einer enteralen Darreichungsform:

A) Man löst 722 mg (1 mMol) des in Beispiel 6 a) beschriebenen Komplexes und 2,423 g (20 mMol) Trishydroxy methylamminomethan in 5 ml Wasser p.i. und füllt auf ein Volumen von 10 ml mit Wasser p.i. auf.

B) Man mischt 45 g Mannit und 20 g Methylhydroxyethylcellulose ca. 3 Minuten, gibt die Mischung durch ein Sieb mit 0,8 mm Maschenweite und mischt nochmals 3 Minuten. Danach wird das erhaltene Pulver mit der nach A) hergestellten Granulierlösung portionsweise befeuchtet und angerieben, das feuchte Granulat durch ein Sieb mit 1,25 mm Maschenweise gegeben, danach 2 Stunden bei 50°C und 27 kPa (200 Torr) getrocknet, durch ein Sieb mit 1,0 mm Maschenweite egalisiert und abschliessend wiederum 3 Minuten gemischt. Das erhaltene Granulat wird zum Gebrauch mit 1 Liter Wasser p.i. vermischt und innerhalb 5 Minuten nach Herstellung verwendet.

*Beispiel 17*

Herstellung von mit Gadolinium-Komplex beladenen Liposomen:

Entsprechend der Vorschrift in Proc. Natl. Acad. Sci. USA 75, 4194 wird ein Lipid-Gemisch, bestehend aus 85 Mol-% Ei-Phosphatidylcholin und 25 Mol-% Cholesterol als Trockensubstanz hergestellt. 500 mg werden anschliessend in 30 ml Diethylether gelöst, und die Lösung unter Ultrabeschallung tropfenweise mit einer Lösung der in Beispiel 6 a) beschriebenen Komplexverbindung versetzt. Man setzt die Ultrabeschallung noch 10 Minuten fort und bringt im Rotavapor zur Trockne. Der gelartige Rückstand wird bei 0°C in 0,125 molarer Kochsalzlösung suspendiert und durch wiederholtes Zentrifugieren (20 000 g/29 Minuten) von nicht verkapseltem Kontrastmittel befreit. Abschliessend wird in Multivials abgefüllt und gefriergetrocknet:

**Patentansprüche**

1. Verbindungen der allgemeinen Formel I

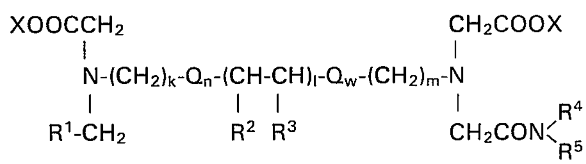

(I),

worin

$R^1$ eine COOX-Gruppe oder die Gruppe 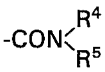 bedeutet,

$R^2$ und $R^3$ gleich oder verschieden sind und ein Wasserstoffatom, einen niederen gerad- oder verzweigtkettigen Kohlenwasserstoffrest, eine Phenyl- oder eine Benzylgruppe oder $R^2$ und $R^3$ zusammen einen Trimethylen- oder Tetramethylenrest bedeuten,

$R^4$ einen gerad- oder verzweigtkettigen Mono- oder Polyhydroxyalkylrest,

$R^5$ ein Wasserstoffatom, einen niederen gerad- oder verzweigtkettigen gegebenenfalls mono oder polyhydroxylierten Kohlenwasserstoffrest darstellen,

Q die Gruppe $-N-(CH_2)_j-R^X$, wobei j die Ziffern 1 bis 4 und $R^x$ eine COOX-Gruppe oder, wenn $j > 1$, die

Gruppe 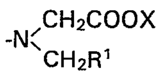 bedeuten,

wobei k und m gleich oder verschieden sind und die Ziffern 0 oder 2 darstellen,

l die Ziffern 1 oder 2 bedeuten und n und w gleich oder verschieden sind und die Zahlen 0 oder 1 bedeuten,

und n und w nur dann die Zahl 1 darstellen, wenn k und m die Zahl 2 bedeuten, und

X Wasserstoffatome und/oder Metallionenäquivalente darstellen,

sowie deren Salze mit physiologisch unbedenklichen Basen.

2. Verbindungen der allgemeinen Formel Ia

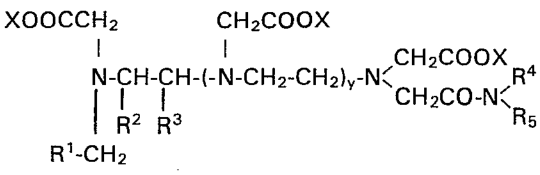

(Ia),

worin

X, $R^1$, $R^2$, $R^3$, $R^4$ und $R^5$ die in Anspruch 1 genannte Bedeutung besitzen und

y die Ziffern 0, oder falls $R_2$ und $R_3$ Wasserstoffatome sind auch 1 oder 2 darstellen,

sowie deren Salze mit physiologisch unbedenklichen Basen.

3. Verbindungen der allgemeinen Formel I und Ia gemäss Patentanspruch 1 und 2, dadurch gekennzeichnet, dass X Wasserstoffatome darstellen.

4. $N^6$-Carboxymethyl-$N^3$,$N^9$-bis(2,3,4-trihydroxybutyl-carbamoylmethyl)-3,6,9-triazaundecandisäure,

$N^6$-Carboxymethyl-$N^3$,$N^9$-bis(2,3-dihydroxypropyl-carbamoylmethyl)-3,6,9-triazaundecandisäure,

$N^6$-Carboxymethyl-$N^3$,$N^9$-bis(2,3-dihydroxypropyl-N-methyl-carbamoylmethyl)-3,6,9-triazaundecandisäure,

$N^6$-Carboxymethyl-$N^3$,$N^9$-bis[2-hydroxy-1-(hydroxymethyl)-ethyl-carbamoylmethyl]-3,6,9-triazaundecandisäure,

$N^6$-Carboxymethyl-$N^3$,$N^9$-bis[bis(2-hydroxyethyl)-carbamoylmethyl]-3,6,9-triazaundecandisäure,

$N^6$-Carboxymethyl-$N^3$,$N^9$-bis(2,3,4,5,6-pentahydroxyhexyl-N-methyl-carbamoylmethyl)-3,6,9-triazaundecandisäure,

$N^6$-Carboxymethyl-$N^3$,$N^9$-bis[2,3-dihydroxypropyl-1-(hydroxymethyl)-carbamoylmethyl]-3,6,9-triazaundecandisäure.

5. trans-1,2-Diamino-N,N'-bis(carboxymethyl)-N,N'-bis(2,3-dihydroxypropyl-carbamoylmethyl)-cyclohexan,

trans-1,2-Diamino-N,N'-bis(carboxymethyl)-N,N'-bis(2,3-dihydroxypropyl-N-methyl-carbamoylmethyl)-cyclohexan,

trans-1,2-Diamino-N,N'-bis(carboxymethyl)-N,N'-bis[bis(2-hydroxyethyl)-carbamoylmethyl]-cyclohexan,

trans-1,2-Diamino-N,N'-bis(carboxymethyl)-N,N'-bis(2-hydroxy-ethyl-carbamoylmethyl)-cyclohexan,

trans-1,2-Diamino-N,N'-bis(carboxymethyl)-N,N'-bis[2-hydroxy-1-(hydroxymethyl)-ethyl-carbamoylmethyl]-cyclohexan.

6. $N^3$,$N^6$-Bis(2,3-dihydroxypropyl-N-methyl-carbamoylmethyl)-3,6-diazaoctandisäure,

$N^3$,$N^6$-Bis[2-hydroxy-1-(hydroxymethyl)-ethyl-carbamoylmethyl]-3,6-diazaoctandisäure,

$N^3$,$N^6$-Bis(2,3-dihydroxy-propyl-carbamoylmethyl)-3,6-diazaoctandisäure,

$N^3$,$N^6$-Bis[bis(2-hydroxyethyl)-carbamoylmethyl]-3,6-diazaoctandisäure,

$N^3$,$N^6$-Bis[2,3-dihydroxypropyl-1-(hydroxymethyl)-carbamoylmethyl]-3,6-diazaoctandisäure,

$N^3$,$N^6$-Bis(2-hydroxyethyl-carbamoylmethyl)-3,6-diazaoctandisäure.

7. Verwendung der Verbindungen gemäss Patentanspruch 3 bis 6 als Komplexbildner.

8. Verfahren zur Herstellung von Verbindungen der allgemeinen Formel I gemäss Anspruch 1, dadurch gekennzeichnet, dass man in an sich bekannter Weise

a) zur Herstellung von Verbindungen in denen $R^1$ die Gruppe

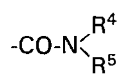 bedeutet,

entweder

eine Verbindung der allgemeinen Formel II

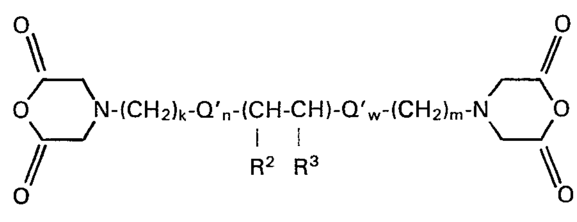

(II),

worin

$R^2$ und $R^3$ die in Anspruch 1 genannte Bedeutung besitzen

und Q' die in Anspruch 1 genannte Bedeutung für Q besitzt, jedoch anstelle der Gruppe

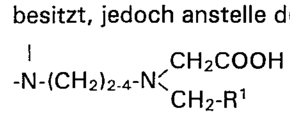

die Gruppe $-\overset{|}{N}-(CH_2)_{2-4}-N$ 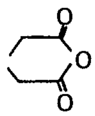 bedeutet

und k, n, l, w und m die in Anspruch 1 genannten Bedeutungen haben,

mit einem Aminoalkohol der allgemeinen Formel

$$HN\begin{cases}R^4\\R^5\end{cases}$$

umsetzt
oder eine Verbindung der allgemeinen Formel III

$$\begin{matrix}HOOCCH_2\\R^6OOCCH_2\end{matrix}\rangle N-(CH_2)_k-Q''_n-(\underset{R^2}{\underset{|}{CH}}-\underset{R^3}{\underset{|}{CH}})_l-Q''_w-(CH_2)_m-N\langle\begin{matrix}CH_2COOH\\CH_2COOR^6\end{matrix} \quad (III),$$

worin
$R^2$ und $R^3$ die in Anspruch 1 genannte Bedeutung haben,
$R^6$ für einen niederen Alkylrest steht und Q'' die in Anspruch 1 genannte Bedeutung besitzt, jedoch statt der Gruppe

$$-N-(CH_2)_{2-4}-N\langle\begin{matrix}CH_2COOH\\CH_2-R^1\end{matrix}$$

die Gruppe $-N-(CH_2)_{2-4}-N\langle\begin{matrix}CH_2COOH\\CH_2COOR^6\end{matrix}$ bedeutet mit

$R^6$ in der oben genannten Bedeutung, und k, n, l, w und m die in Anspruch 1 genannten Bedeutungen haben,
mit einem Aminoalkohol der allgemeinen Formel

$$HN\begin{cases}R^4\\R^5\end{cases}$$

umsetzt
oder
b) zur Herstellung von Verbindungen der Formel I, in denen
$R^1$ eine Carboxylgruppe bedeutet,
eine Verbindung der allgemeinen Formel IV

$$(HOOCCH_2)_2N-(CH_2)_k-Q_n-(\underset{R^2}{\underset{|}{CH}}-\underset{R^3}{\underset{|}{CH}})-Q_w-(CH_2)_m-N\langle\begin{matrix}CH_2COY\\CH_2COZ\end{matrix} \quad (IV),$$

worin
Y und Z gemeinsam ein Sauerstoff-Atom oder Y eine Hydroxygruppe und Z die Gruppierung $OR^6$ darstellen und
$R^2$, $R^3$, $R^6$, k, n, w und m die oben genannten Bedeutungen besitzen, mit einem Aminoalkohol der allgemeinen Formel

$$HN\begin{cases}R^4\\R^5\end{cases}$$

umsetzt
und gewünschtenfalls aus den Säuren die Salze, Metallkomplexe oder Metallkomplexsalze herstellt.

9. Verbindungen gemäss Patentanspruch 1 und 2, dadurch gekennzeichnet, dass mindestens einer der SubstituentenX ein Alkalimetallion darstellt.

10. Verbindungen gemäss Patentanspruch 1, 2 und 9, dadurch gekennzeichnet, dass zumindest ein Teil der Substituenten X Ionenäquivalente von Metallen der Ordungszahlen 20 bis 32, 42 bis 44, 49 oder 57 bis 83 sind.

11. Gadolinium-Komplexe von $N^6$-Carboxymethyl-$N^3$,$N^9$-bis(2,3,4-trihydroxybutyl-carbamoylmethyl)-3,6,9-triazaundecandisäure, $N^6$-Carboxymethyl-$N^3$,$N^9$-bis(2,3-dihydroxypropyl-carbamoylmethyl)3,6,9-triazaundecandisäure, $N^6$-Carboxymethyl-$N^3$,$N^9$-bis(2,3-dihydroxypropyl-N-methyl-carbamoylmethyl)-3,6,9-triazaundecandisäure, $N^6$-Carboxymethyl-$N^3$,$N^9$-bis[2-hydroxy-1-(hydroxymethyl)-ethyl-carbamoylmethyl]-3,6,9-triazaundecandisäure, $N^6$-Carboxymethyl-$N^3$,$N^9$-bis[bis(2-hydroxyethyl)-carbamoylmethyl]-3,6,9-triazaundecandisäure, $N^6$-Carboxymethyl-$N^3$,$N^9$-bis(2,3,4,5,6-pentahydroxyhexyl-N-methyl-carbamoylmethyl)-3,6,9-triazaundecandisäure und $N^6$-Carboxymethyl-$N^3$,$N^9$-bis[2,3-dihydroxypropyl-1-(hydroxymethyl)-carbamoylmethyl]-3,6,9-triazaundecandisäure.

12. Lanthan-, Dysprosium-, Holmium-, Terbium-, Ytterbium-, Cer-, Samarium-, Wismut-, Chrom- und Eisen-Komplex von $N^6$-Carboxymethyl-$N^3$,$N^9$-bis-(2,3-dihydroxypropyl-N-methyl-carbamoylmethyl)-3,6,9-triazaundecandisäure.

13. Mangan-Komplexe von trans-1,2-Diamino-N,N'-bis(carboxymethyl)-N,N'-bis(2,3-dihydroxypropyl-carbamoylmethyl)-cyclohexan, trans-1,2-Diamino-N,N'-bis(carboxymethyl)-N,N'-bis(2,3-dihydroxypropyl-N-methyl-carbamoylmethyl)-cyclohexan, trans-1,2-Diamino-N,N'-bis(carboxymethyl)-N,N'-bis[bis(2-hydroxyethyl)-carbamoylmethyl]-cyclohexan, trans-1,2-Diamino-N,N'-bis(carboxymethyl)-N,N'-bis(2-hydroxy-ethyl-carbamoylmethyl)-cyclohexan, trans-1,2-Diamino-N,N'-bis(carbamoylmethyl)-N,N'-bis[2-hydroxy-1-(hydroxymethyl)-ethyl-carbamoylmethyl]-cyclohexan und $N^3$,-$N^6$-bis(2,3-dihydroxypropyl-N-methyl-carbamoylmethyl)-3,6-diazaoctandisäure.

14. Natriumsalze des Calciumkomplexes der $N^6$-Carboxymethyl-$N^3$,$N^9$-bis(2,3-dihydroxypropyl-carbamoylmethyl)-3,6,9-triazaundecandisäure.

15. Komplexe der $N^6$-Carboxymethyl-$N^3$,$N^9$-bis-(2,3-dihydroxypropyl-carbamoylmethyl)-3,6,9-triazaundecandisäure mit $^{111}In$, $^{113}In$, $^{67}Cu$, $^{67}Ga$, $^{169}Yb$, $^{153}Gd$ und $^{99m}Tc$.

16. Mono-N-methylglucaminsalz des Zink-Komplexes der $N^6$-Carboxymethyl-$N^3$,$N^9$-bis(2,3-dihy

droxypropyl-N-methyl-carbamoylmethyl)-3,6,9-triazaundecandisäure.

17. Diagnostische Mittel enthaltend eine Verbindung gemäss den Patentansprüchen 11 bis 13 und 15.

18. Pharmaceutische Präparate enthaltend eine Verbindung gemäss Patentansprüchen 14 und 16.

19. Verwendung von mindestens einem physiologisch verträglichen Komplexsalz der allgemeinen Formel I

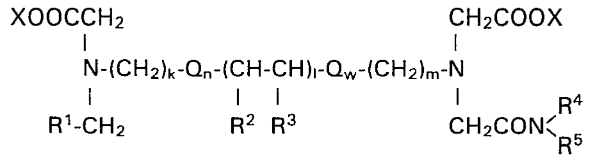

(I),

worin

$R^1$ eine COOX-Gruppe oder die Gruppe $-CON\genfrac{}{}{0pt}{}{R^4}{R^5}$ bedeutet,

$R^2$ und $R^3$ gleich oder verschieden sind und ein Wasserstoffatom, einen niederen gerad- oder verzweigtkettigen Kohlenwasserstoffrest, eine Phenyl- oder eine Benzylgruppe oder entweder $R^2$ oder $R^3$

die Gruppe $-N\genfrac{}{}{0pt}{}{CH_2COOX}{CH_2\text{-}R^1}$

oder $R^2$ und $R^3$ zusammen einen Trimethylen- oder Tetramethylenrest bedeuten,

$R^4$ einen gerad- oder verzweigtkettigen Mono- oder Polyhydroxyalkylrest,

$R^5$ ein Wasserstoffatom, einen niederen gerad- oder verzweigtkettigen gegebenenfalls mono- oder polyhydroxylierten Kohlenwasserstoffrest darstellen,

Q ein Sauerstoff- oder Schwefelatom oder die Gruppe $-N\text{-}(CH_2)_j\text{-}R^x$, wobei j die Ziffern 1 bis 4 und $R^x$ ein Wasserstoffatom, eine niedere Alkoxygruppe mit 1 bis 4 Kohlenstoffatomen, eine COOX-Gruppe oder,

wenn $j > 1$, die Gruppe $-N\genfrac{}{}{0pt}{}{CH_2COOX}{CH_2R^1}$ bedeuten,

wobei k und m gleich oder verschieden sind und die Ziffern 0 oder 2 darstellen,

l die Ziffern 1 oder 2 bedeuten und n und w gleich oder verschieden sind und die Zahlen 0 oder 1 bedeuten,

wobei n und w nur dann die Zahl 1 darstellen, wenn k und m die Zahl 2 bedeuten, und

X Wasserstoffatome und/oder Metallionenäquivalente darstellen,

sowie deren Salze mit physiologisch unbedenklichen organischen Basen,

dadurch gekennzeichnet, dass zumindest ein Teil der Substituenten X Ionenäquivalente von Metallen der Ordnungszahlen 20 bis 32, 42 bis 44, 49 oder 57 bis 83 sind,

zur Herstellung von diagnostischen Mitteln.

20. Verwendung von Gadolinium-Komplexen von $N^6$-Carboxymethyl-$N^3$,$N^9$-bis(2,3,4-trihydroxybutyl-carbamoylmethyl)-3,6,9-triazaundecandisäure, $N^6$-Carboxymethyl-$N^3$,$N^9$-bis(2,3-dihydroxypropyl-carbamoylmethyl)3,6,9-triazaundecandisäure, $N^6$-Carboxymethyl-$N^3$,$N^9$-bis(2,3-dihydroxypropyl-N-methyl carbamoylmethyl)-3,6,9-triazaundecandisäure, $N^6$-Carboxymethyl-$N^3$,$N^9$-bis[2-hydroxy-1-(hydroxymethy)-ethyl-carbamoylmethyl]-3,6,9-triazaundecandisäure, $N^6$-Carboxymethyl-$N^3$,$N^9$-bis-[bis(2-hydroxyethyl)-carbamoylmethyl]-3,6,9-triazaundecandisäure, $N^6$-Carboxymethyl-$N^3$,$N^9$-bis-(2,3,4,5,6-pentahydroxyhexyl-N-methyl-carbamoylmethyl)-3,6,9-triazaundecandisäure und $N^6$-Carboxymethyl-$N^3$,$N^9$-bis[2,3-dihydroxypropyl-1-(hydroxymethyl)-carbamoylmethyl]-3,6,9-triazaundecandisäure zur Herstellung von diagnostischen Mitteln.

21. Verwendung von Lanthan-, Dysprosium-, Holmium-, Terbium-, Ytterbium-, Cer-, Samarium-, Wismut-, Chrom und Eisen-Komplexen von $N^6$-Carboxymethyl-$N^3$,$N^9$-bis(2,3-dihydroxypropyl-N-methyl-carbamoylmethyl)-3,6,9-triazaundecandisäure

zur Herstellung von diagnostischen Mitteln.

22. Verwendung von Mangan-Komplexen von trans-1,2-Diamino-N,N'-bis(carboxymethyl)-N-N'-bis(2,3-dihydroxypropyl-carbamoylmethyl)-cyclohexan, trans-1,2-Diamino-N,N'-bis(carboxymethyl)-N-N'-bis(2,3-dihydroxypropyl-N-methyl-carbamoylmethyl)-cyclohexan, trans-1,2-Diamino-N,N'-bis(carboxymethyl)-N-N'-bis[bis(2-hydroxyethyl)-carbamoylmethyl]-cyclohexan, trans-1,2-Diamino-N,N'-bis(carboxymethyl)-N-N'-bis(2-hydroxy-ethyl-carbamoylmethyl)-cyclohexan, trans-1,2-Diamino-N,N'-bis(carboxymethyl)-N-N'-bis[2-hydroxy-1-(hydroxymethyl)-ethyl-carbamoylmethyl]-cyclohexan und $N^3$,$N^6$-Bis(2,3-dihydroxypropyl-N-methyl-carbamoylmethyl)-3,6-diazaoctandisäure

zur Herstellung von diagnostischen Mitteln.

23. Verwendung von Natriumsalz des Calciumkomplexes der $N^6$-Carboxymethyl-$N^3$,$N^9$-bis(2,3-dihydroxypropyl-carbamoylmethyl)-3,6,9-triazaundecandisäure

zur Herstellung von diagnostischen Mitteln.

24. Verwendung von Komplexen der $N^6$-Carboxymethyl-$N^3$,$N^9$-bis(2,3-dihydroxypropyl-carbamoylmethyl)-3,6,9-triazaundecandisäure mit $^{111}In$, $^{113m}In$, $^{67}Cu$, $^{67}Ga$, $^{169}Yb$, $^{153}Gd$ und $^{99m}Tc$

zur Herstellung von diagnostischen Mitteln.

25. Verwendung von Mono-N-methylglucaminsalz des Zink-Komplexes der $N^6$-Carboxymethyl-$N^3$,$N^9$-bis(2,3-dihydroxy-N-methyl-carbamoylmethyl)-3,6,9-triazaundecandisäure

zur Herstellung von diagnostischen Mitteln.

**Claims**

1. Compounds of the general formula I

```
XOOCCH2                                 CH2COOX
   |                                       |
   N-(CH2)k-Qn-(CH-CH)l-Qw-(CH2)m-N
   |             |   |                     |     R4
R1-CH2           R2  R3                 CH2CON<
                                                 R5
```
(I),

where $R^1$ represents a COOX group or the group

$-CON<^{R^4}_{R^5}$

$R^2$ and $R^3$ are the same or different and represent a hydrogen atom, a lower straight or branched chain hydrocarbon group, a phenyl or a benzyl group, or $R^2$ and $R^3$ together represent a trimethylene or tetramethylene group,
$R^4$ represents a straight or branched chain mono- or polyhydroxyalkyl group,
$R^5$ represents a hydrogen atom, a lower straight or branched chain optionally mono- or polyhydroxylated hydrocarbon group,
Q represents the group $-N-(CH_2)_j-R^x$, where j is a number 1 to 4 and $R^x$ represents a COOX group or, when j > 1, the group

$-N<^{CH_2COOX}_{CH_2R^1}$

where k and m are the same or different and represent the numbers 0 or 2,
l represents the numbers 1 or 2 and n and w are the same or different and represent the numbers 0 or 1, where n and w only represent the number 1 when k and m represent the number 2, and
X represents hydrogen and/or metal ion equivalents, as well as their salts with physiologically acceptable bases.

2. Compound of the general formula Ia

```
XOOCCH2          CH2COOX
   |               |           CH2COOX
   N-CH-CH-(-N-CH2-CH2)y-N<           R4
   |  |   |                    CH2CO-N<
   |  R2  R3                          R5
R1-CH2
```
(Ia),

wherein X, $R^1$, $R^2$, $R^3$, $R^4$ and $R^5$ have the meanings given in claim 1 and
y represents 0 or, in the case that $R_2$ and $R_3$ are hydrogen atoms, also 1 or 2
as well as their salts with physiologically acceptable bases.

3. Compounds of general formula I and Ia according to claims 1 and 2, characterised in that X represents hydrogen.

4. $N^6$-carboxymethyl-$N^3$,$N^9$-bis(2,3,4-trihydroxybutyl-carbamoylmethyl)-3,6,9-triazaundecane dicarboxylic acid, $N^6$-carboxymethyl-$N^3$,$N^9$-bis(2,3-dihydroxypropyl-carbamoylmethyl)-3,6,9-triazaundecane dicarboxylic acid, $N^6$-carboxymethyl-$N^3$,$N^9$-bis(2,3-dihydroxypropyl-N-methylcarbamoylmethyl)-3,6,9-triazaundecane dicarboxylic acid, $N^6$-carboxymethyl-$N^3$,$N^9$-bis[2-hydroxy-1-(hydroxymethyl)-ethylcarbamoylmethyl]-3,6,9-triazaundecane dicarboxylic acid, $N^6$-carboxymethyl-$N^3$,$N^9$-bis[bis(2-hydroxyethyl)-carbamoylmethyl]-3,6,9-triazaundecane dicarboxylic acid, $N^6$-carboxymethyl-$N^3$,$N^9$-bis(2,3,4,5,6-pentahydroxyhexyl-N-methylcarbamoylmethyl)-3,6,9-triazaundecane dicarboxylic acid, $N^6$-carboxymethyl-$N^3$,$N^9$-bis[2,3-dihydroxypropyl-1-(hydroxymethyl)carbamoylmethyl]-3,6,9-triazaundecane dicarboxylic acid.

5. Trans-1,2-diamino-N,N'-bis(carboxymethyl)-N-N'-bis(2,3-dihydroxypropylcarbamoylmethyl)-cyclohexane, trans-1,2-diamino-N,N'-bis(carboxymethyl)-N-N'-bis(2,3-dihydroxypropyl-N-methyl-carbamoylmethyl)cyclohexane, trans-1,2-diamino-N,N'-bis(carboxymethyl)-N,N'-bis[bis(2-hydroxyethyl)carbamoylmethyl]cyclohexane, trans-1,2-diamino-N,N'-bis(carboxymethyl)-N-N'-bis(2-hydroxyethylcarbamoylmethyl)cyclohexane, trans-1,2-diamino-N,N'-bis(carboxymethyl)-N-N'-bis[2-hydroxy-1-(hydroxymethyl)ethylcarbamoylmethyl]cyclohexane.

6. $N^3$,$N^6$-bis(2,3-dihydroxypropyl-N-methylcarbamoylmethyl)-3,6-diazaoctane dicarboxylic acid, $N^3$,$N^6$-bis[2-hydroxy-1-(hydroxymethyl)ethylcarbamoylmethyl)-3,6-diazaoctane dicarboxylic acid, $N^3$,$N^6$-bis(2,3-dihydroxypropylcarbamoylmethyl)-3,6-diazaoctane dicarboxylic acid, $N^3$,$N^6$-bis[bis(2-hydroxyethyl)carbamoylmethyl)-3,6-diazaoctane dicarboxylic acid, $N^3$,$N^6$-bis[2,3-dihydroxypropyl-1-(hydroxymethyl)carbamoylmethyl]-3,6-diazaoctane dicarboxylic acid, $N^3$,$N^6$-bis(2-hydroxyethylcarbamoylmethyl]-3,6-diazaoctane dicarboxylic acid.

7. Use of the compounds according to claims 3 to 6 as complex formers.

8. Process for the preparation of compounds of general formula I according to claim 1, characterised in that one reacts in a known manner

a) for the preparation of compounds in which $R^1$ represents the group

$-CON<^{R^4}_{R^5}$

either a compound of the general formula II

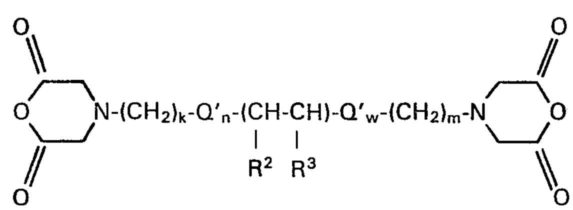

(II),

where $R^2$ and $R^3$ have the meanings given in claim 1 and Q' has the meaning given in claim 1 for Q, however instead of the group

$-N-(CH_2)_{2-4}-N<^{CH_2COOH}_{CH_2-R^1}$

the group

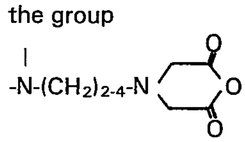

is meant

and k, n, l, w and m have the meanings given in claim 1, with an aminoalcohol of the general formula

$$HN\begin{matrix}R^4\\R^5\end{matrix}$$

or a compound of the general formula III

$$\begin{matrix}HOOCCH_2\\R^6OOCCH_2\end{matrix}>N-(CH_2)_k-Q''_n-\underset{R^2}{\underset{|}{CH}}-\underset{R^3}{\underset{|}{CH}})_l-Q''_w-(CH_2)_m-N<\begin{matrix}CH_2COOH\\CH_2COOR^6\end{matrix} \quad (III),$$

where $R^2$ and $R^3$ have the meanings given in claim 1, $R^6$ represents a lower alkyl group and Q'' has the meaning given in claim 1, but instead of the group

$$-N-(CH_2)_{2-4}-N<\begin{matrix}CH_2COOH\\CH_2-R^1\end{matrix}$$

the group

$$-N-(CH_2)_{2-4}-N<\begin{matrix}CH_2COOH\\CH_2COOR^6\end{matrix}$$

is meant with $R^6$ having the above given meaning, and k, n, l, w and m having the meanings given in claim 1, with an aminoalcohol of the general formula

$$HN\begin{matrix}R^4\\R^5\end{matrix}$$

or

b) for the preparation of compounds of formula I in which

$R^1$ represents a carboxyl group,
a compound of the general formula

$$(HOOCCH_2)_2N-(CH_2)_k-Q_n-\underset{R^2}{\underset{|}{CH}}-\underset{R^3}{\underset{|}{CH}})-Q_w-(CH_2)_m-N<\begin{matrix}CH_2COY\\CH_2COZ\end{matrix} \quad (IV),$$

where Y and Z are each oxygen or Y represents a hydroxy group and Z represents an $OR^6$ grouping, and $R^2$, $R^3$, $R^6$, k, n, w and m have the above given meanings, with an aminoalcohol of the general formula

$$HN\begin{matrix}R^4\\R^5\end{matrix}$$

and if desired from the acids the salts, metal complexes or metal complex salts are prepared.

9. Compounds according to claims 1 and 2, characterised in that at least one of the substituents X represents an alkali-metal ion.

10. Compounds according to claims 1, 2 and 9, characterised in that at least one part of the substituents X are ion-equivalents of metals of atomic numbers 20 to 32, 42 to 44, 49 or 57 to 83.

11. Gadolinium complexes of
$N^6$-carboxymethyl-$N^3$,$N^9$-bis(2,3,4-trihydroxybutyl-carbamoylmethyl)-3,6,9-triazaundecane dicarboxylic acid,
$N^6$-carboxymethyl-$N^3$,$N^9$-bis(2,3-dihydroxypropyl-carbamoylmethyl)-3,6,9-triazaundecane dicarboxylic acid,
$N^6$-carboxymethyl-$N^3$,$N^9$-bis(2,3-dihydroxypropyl-N-methylcarbamoylmethyl)-3,6,9-triazaundecane dicarboxylic acid,
$N^6$-carboxymethyl-$N^3$,$N^9$-bis[2-hydroxy-1-(hydroxymethyl)-ethylcarbamoylmethyl]-3,6,9-triazaundecane dicarboxylic acid,
$N^6$-carboxymethyl-$N^3$,$N^9$-bis[bis(2-hydroxyethyl)-carbamoylmethyl]-3,6,9-triazaundecane dicarboxylic acid,
$N^6$-carboxymethyl-$N^3$,$N^9$-bis(2,3,4,5,6-pentahydroxyhexyl-N-methylcarbamoylmethyl)-3,6,9-triazaundecane dicarboxylic acid, and
$N^6$-carboxymethyl-$N^3$,$N^9$-bis[2,3-dihydroxypropyl-1-(hydroxymethyl)carbamoylmethyl-3,6,9-triazaundecane dicarboxylic acid.

12. Lanthanum-, dysprosium-, holmium-, terbium-, ytterbium-, cerium-, samarium-, bismuth-, chromium- and iron complex of $N^6$-carboxymethyl-$N^3$,$N^9$-bis(2,3-dihydroxypropyl-N-methylcarbamoylmethyl)-3,6,9-triazaundecane dicarboxylic acid.

13. Manganese complex of
trans-1,2-diamino-N,N'-bis(carboxymethyl)-N,N'-bis(2,3-dihydroxypropylcarbamoylmethyl)cyclohexane,
trans-1,2-diamino-N,N'-bis(carboxymethyl)-N,N'-bis(2,3-dihydroxypropyl-N-methylcarbamoylmethyl)cyclohexane,
trans-1,2-diamino-N,N'-bis(carboxymethyl)-N,N'-bis[bis(2-hydroxyethyl)-carbamoylmethyl)cyclohexane,
trans-1,2-diamino-N,N'-bis(carboxymethyl)-N,N'-bis(2-hydroxyethylcarbamoylmethyl)cyçlohexane,
trans-1,2-diamino-N,N'-bis(carboxymethyl)-N,N'-bis[2-hydroxy-1-(hydroxymethyl)ethylcarbamoylmethyl]cyclohexane, and
$N^3$,$N^6$-bis(2,3-dihydroxypropyl-N-methylcarbamoylmethyl)-3,6-diazaoctane dicarboxylic acid.

14. Sodium salt of the calcium complex of $N^6$-carboxymethyl-$N^3$,$N^9$-bis(2,3-dihydroxypropylcarbamoylmethyl)-3,6,9-triazaundecane dicarboxylic acid.

15. Complexes of $N^6$-carboxymethyl-$N^3$,$N^9$-bis-

(2,3-dihydroxypropylcarbamoylmethyl)-3,6,9-triazaundecane dicarboxylic acid with $^{111}In$, $^{113m}In$, $^{67}Cu$, $^{67}Ga$, $^{169}Yb$, $^{153}Gd$ and $^{99m}Tc$.

16. Mono-N-methylglucamin salt of the zinc complex of $N^6$-carboxymethyl-$N^3$,$N^9$-bis(2,3-dihydroxypropyl-N-methylcarbamoylmethyl)-3,6,9-triazaundecane dicarboxylic acid.

17. Diagnostic agent containing a compound according to claims 11 to 13 and 15.

18. Pharmaceutical preparations containing a compound according to claims 14 and 16.

19. Use of at least one physiologically compatible complex salt of the general formula I

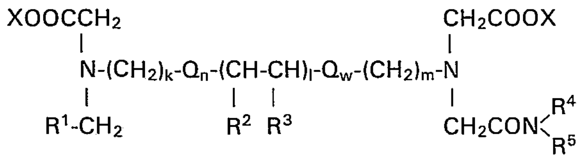

(I),

where $R^1$ represents a COOX group or the group

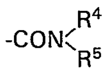

$R^2$ and $R^3$ are the same or different and represent a hydrogen atom, a lower straight or branched chain hydrocarbon group, a phenyl or a benzyl group, or either $R^2$ or $R^3$ represents the group

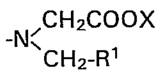

or $R^2$ and $R^3$ together represent a trimethylene or tetramethylene group,
$R^4$ represents a straight or branched chain mono- or polyhydroxyalkyl group,
$R^5$ represents a hydrogen atom, a lower straight or branched chain optionally mono- or polyhydroxylated hydrocarbon group,
Q represents an oxygen or sulphur atom or the group $-N-(CH_2)_j-R^x$, where j is a number 1 to 4 and $R^x$ represents a hydrogen atom, a lower alkyl group of 1 to 4 carbon atoms, a COOX group or, when $j > 1$, the group

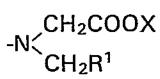

where k and m are the same or different and represent the numbers 0 or 2,
l represents the numbers 1 or 2 and n and w are the same or different and represent the numbers 0 or 1, where n and w only represent the number 1 when k and m represent the number 2, and
X represents hydrogen and/or metal ion equivalents, as well as their salts with physiologically acceptable bases,
characterised in that at least one part of the substituents X are ion-equivalents of metals of atomic numbers 20 to 32, 42 to 44, 49 or 57 to 83, in the preparation of diagnostic agents.

20. Use of the gadolinium complexes of
$N^6$-carboxymethyl-$N^3$,$N^9$-bis(2,3,4-trihydroxybutyl-carbamoylmethyl)-3,6,9-triazaundecane dicarboxylic acid,
$N^6$-carboxymethyl-$N^3$,$N^9$-bis(2,3-dihydroxypropyl-carbamoylmethyl)-3,6,9-triazaundecane dicarboxylic acid,
$N^6$-carboxymethyl-$N^3$,$N^9$-bis(2,3-dihydroxypropyl-N-methylcarbamoylmethyl)-3,6,9-triazaundecane dicarboxylic acid,
$N^6$-carboxymethyl-$N^3$,$N^9$-bis[2-hydroxy-1-hydroxymethyl)-ethylcarbamoylmethyl]-3,6,9-triazaundecane dicarboxylic acid,
$N^6$-carboxymethyl-$N^3$,$N^9$-bis[bis(2-hydroxyethyl)-carbamoylmethyl]-3,6,9-triazaundecane dicarboxylic acid,
$N^6$-carboxymethyl-$N^3$,$N^9$-bis(2,3,4,5,6-pentahydroxyhexyl-N-methylcarbamoylmethyl)-3,6,9-triazaundecane dicarboxylic acid, and
$N^6$-carboxymethyl-$N^3$,$N^9$-bis[2,3-dihydroxypropyl-1-(hydroxymethyl)carbamoylmethyl]-3,6,9-triazaundecane dicarboxylic acid,
in the preparation of diagnostic agents.

21. Use of lanthanum, dysprosium-, holmium-, terbium-, ytterbium-, cerium-, samarium-, bismuth-, chromium- and iron complex of $N^6$-carboxymethyl-$N^3$,$N^9$-bis(2,3-dihydroxypropyl-N-methylcarbamoylmethyl)-3,6,9-triazaundecane dicarboxylic acid
in the preparation of diagnostic agents.

22. Use of manganese complexes of
trans-1,2-diamino-N,N'-bis(carboxymethyl)-N,N'-bis(2,3-dihydroxypropylcarbamoylmethyl)-cyclohexane,
trans-1,2-diamino-N,N'-bis(carboxymethyl)-N,N'-bis(2,3-dihydroxypropyl-N-methylcarbamoylmethyl)-cyclohexane,
trans-1,2-diamino-N,N'-bis(carboxymethyl)-N,N'-bis[bis(2-hydroxyethyl)carbamoylmethyl]cyclohexane,
trans-1,2-diamino-N,N'-bis(carboxymethyl)-N,N'-bis(2-hydroxyethylcarbamoylmethyl)cyclohexane,
trans-1,2-diamino-N,N'-bis(carboxymethyl)-N,N'-bis[2-hydroxy-1-(hydroxymethyl)ethylcarbamoylmethyl]cyclohexane, and
$N^3$,$N^6$-bis(2,3-dihydroxypropyl-N-methylcarbamoylmethyl)-3,6-diazaoctane dicarboxylic acid
in the preparation of diagnostic agents.

23. Use of sodium salt of the calcium complex of $N^6$-carboxymethyl-$N^3$,$N^9$-bis(2,3-dihydroxypropylcarbamoylmethyl)-3,6,9-triazaundecane dicarboxylic acid
in the preparation of diagnostic agents.

24. Use of complexes of $N^6$-carboxymethyl-$N^3$,-$N^9$-bis(2,3-dihydroxypropylcarbamoylmethyl)-3,6,-9-triazaundecane dicarboxylic acid with $^{111}In$, $^{113m}In$, $^{67}Cu$, $^{67}Ga$, $^{169}Yb$, $^{153}Gd$ and $^{99m}Tc$
in the preparation of diagnostic agents.

25. Use of mono-N-methylglucamine salt of the zinc complex of $N^6$-carboxymethyl-$N^3$,$N^9$-bis(2,3-dihydroxypropyl-N-methylcarbamoylmethyl)-3,6,-9-triazaundecane dicarboxylic acid
in the preparation of diagnostic agents.

## Revendications

1. Composés de formule générale I

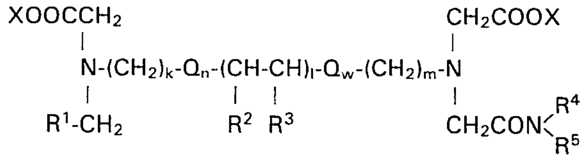

(I),

dans laquelle
$R^1$ représente le groupe COOX ou le groupe

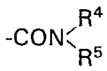

$R^2$ et $R^3$ sont identiques ou différents et représentent chacun un atome d'hydrogène, un reste d'hydrocarbure inférieur linéaire ou ramifié, un groupe phényle ou benzyle, ou bien $R^2$ et $R^3$, pris ensemble, représentent un reste triméthylène ou tétraméthylène,
$R^4$ représente un reste mono- ou poly-hydroxylé, linéaire ou ramifié,
$R^5$ représente un atome d'hydrogène, un reste d'hydrocarbure inférieur linéaire ou ramifié, éventuellement mono- ou poly-hydroxylé,
Q représente le groupe $-N-(CH_2)_j-R^x$, dans lequel j représente les nombres 1 à 4 et $R^x$ représente un groupe COOX, ou bien, quand j est supérieure à 1, il représente le groupe

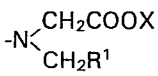

k et m sont identiques ou différents et représentent les chiffres 0 ou 2,
l représente les chiffres 1 ou 2 et
n et w sont identiques ou différents et représentent les nombres 0 ou 1,
n et w ne représentant le nombre 1 que lorsque k et m représentent le nombre 2, et les
X représentent des atomes d'hydrogène et/ou des équivalents d'ions de métaux,
ainsi que leurs sels avec des bases physiologiquement compatibles.

2. Composé répondant à la formule générale Ia

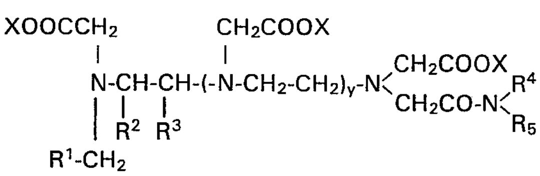

(Ia),

dans laquelle
X, $R^1$, $R^2$, $R^3$, $R^4$ et $R^5$ ont le sens indiqué à la revendication 1,et
y est nul ou, si $R^2$ et $R^3$ représentent des atomes d'hydrogène, y peut également valoir 1 ou 2,
ainsi que leurs sels avec des bases physiologiquement compatibles.

3. Composés de formules générales I et Ia selon les revendications 1 et 2, caractérisés en ce que les X représentent des atomes d'hydrogène.

4. L'acide $N^6$-carboxyméthyl-$N^3$,$N^9$-bis(trihydroxy-2,3,4 butyl-carbamoylméthyl)-triaza-3,6,9 undécanedioïque,
l'acide $N^6$-carboxyméthyl-$N^3$,$N^9$-bis(dihydroxy-2,3 propyl-carbamoylméthyl)-triaza-3,6,9 undécanedioïque,
l'acide $N^6$-carboxyméthyl-$N^3$,$N^9$-bis(dihydroxy-2,3 propyl-N-méthyl-carbamoylméthyl)-triaza-3,6,9 undécanedioïque,
l'acide $N^6$-carboxyméthyl-$N^3$,$N^9$-bis[hydroxy-2-(hydroxyméthyl)-1 éthyl-carbamoylméthyl]-triaza-3,-6,9 undécanedioïque,
l'acide $N^6$-carboxyméthyl-$N^3$,$N^9$-bis[bis-(hydroxy--2-éthyl)-carbamoylméthyl]-triaza-3,6,9 undécanedioïque,
l'acide $N^6$-carboxyméthyl-$N^3$,$N^9$-bis(pentahydroxy--2,3,4,5,6 hexyl-N-méthyl-carbamoylméthyl)-triaza-3,6,9 undécanedioïque,
l'acide $N^6$-carboxyméthyl-$N^3$,$N^9$-bis[dihydroxy-2,3 propyl-(hydroxyméthyl)-1 carbamoylméthyl]-triaza--3,6,9 undécanedioïque.

5. Le trans-diamino-1,2 N,N'-bis(carboxyméthyl)-N,N'-bis(dihydroxy-2,3 propyl-carbamoylméthyl)-cyclohexane,
le trans-diamino-1,2 N,N'-bis(carboxyméthyl)-N,-N'-bis(dihydroxy-2,3 propyl-N-méthyl-carbamoylméthyl)-cyclohexane,
le trans-diamino-1,2 N,N'-bis(carboxyméthyl)-N,-N'-bis[bis(hydroxy-2 éthyl)-carbamoylméthyl]-cyclohexane,
le trans-diamino-1,2 N,N'-bis(carboxyméthyl)-N,-N'-bis(hydroxy-2 éthyl-carbamoylméthyl)-cyclohexane,
le trans-diamino-1,2 N,N'-bis(carboxyméthyl)-N,-N'-bis[hydroxy-2(hydroxyméthyl)-1 éthyl-carbamoylméthyl]-cyclohexane.

6. L'acide $N^3$,$N^6$-bis(dihydroxy-2,3 propyl-N-méthyl-carbamoylméthyl)-diaza-3,6 octanedioïque,
l'acide $N^3$,$N^6$-bis[hydroxy-2(hydroxyméthyl)-1 éthyl-carbamoylméthyl]-diaza-3,6 octanedioïque,
l'acide $N^3$,$N^6$-bis(dihydroxy-2,3 propyl-carbamoylméthyl)-diaza-3,6 octanedioïque,
l'acide $N^3$,$N^6$-bis[bis(hydroxy-2 éthyl)-carbamoylméthyl]-diaza-3,6 octanedioïque,
l'acide $N^3$,$N^6$-bis[dihydroxy-2,3 propyl(hydroxyméthyl)-1 carbamoylméthyl]-diaza-3,6 octanedioïque,
l'acide $N^3$,$N^6$-bis(hydroxy-2 éthyl-carbamoylméthyl)-diaza-3,6 octanedioïque.

7. Utilisation des composés selon les revendications 3 à 6 comme formateurs de complexes.

8. Procédé pour préparer des composés de formule générale I selon la revendication 1, caractérisé en ce que, de façon connue en soi

a) pour préparer des composés dans lesquels $R^1$ représente le groupe

$$-CO-N\begin{matrix}R^4\\R^5\end{matrix}$$

on fait réagier un composé de formule générale II

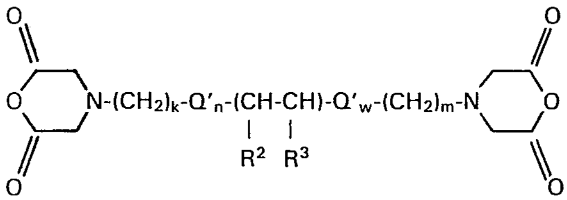

(II),

dans laquelle
$R^2$ et $R^3$ ont le sens indiqué à la revendication 1, et Q' a le sens indiqué pour Q à la revendication 1 mais, au lieu du groupe

$$-\overset{|}{N}-(CH_2)_{2\text{-}4}-N\begin{matrix}CH_2COOH\\CH_2-R^1\end{matrix}$$

il représente le groupe

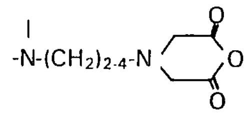

et k, n, l, w et m ont les sens indiqués à la revendication 1, avec un amino-alcool de formule générale

$$HN\begin{matrix}R^4\\R^5\end{matrix}$$

ou bien on fait réagir un composé de formulé générale III

$$\begin{matrix}HOOCCH_2\\R^6OOCCH_2\end{matrix}\!>\!N-(CH_2)_k-Q''_n-(\underset{R^2}{CH}-\underset{R^3}{CH})_l-Q''_w-(CH_2)_m-N\begin{matrix}CH_2COOH\\CH_2COOR^6\end{matrix} \quad (III),$$

dans laquelle
$R^2$ et $R^3$ ont le sens indiqué à la revendication 1, $R^6$ représente un reste alkyle inférieur et Q'' a le sens indiqué pour Q à la revendication 1, mais au lieu du groupe

$$-N-(CH_2)_{2\text{-}4}-N\begin{matrix}CH_2COOH\\CH_2-R^1\end{matrix}$$

il représente le groupe

$$-N-(CH_2)_{2\text{-}4}-N\begin{matrix}CH_2COOH\\CH_2COOR^6\end{matrix}$$

dans lequel

$R^6$ a le sens indiqué ci-dessus, et k, n, l, w et m ont les sens indiqués à la revendication 1, avec un amino-alcool de formule générale

$$HN\begin{matrix}R^4\\R^5\end{matrix}$$

ou

b) pour préparer des composés de formule I, dans lesquels $R^1$ représente un groupe carboxyle, on fait réagir un composé de formule générale IV

$$(HOOCCH_2)_2N-(CH_2)_k-Q_n-(\underset{R^2}{CH}-\underset{R^3}{CH})-Q_w-(CH_2)_m-N\begin{matrix}CH_2COY\\CH_2COZ\end{matrix} \quad (IV),$$

dans laquelle
Y et Z représentent ensemble un atome d'oxygène ou bien Y représente un groupe hydroxy et Z représente le groupement $OR^6$, et
$R^2$, $R^3$, $R^6$, k, n, w et m ont les sens indiqués ci-dessus, avec un amino-alcool de formule générale

$$HN\begin{matrix}R^4\\R^5\end{matrix}$$

et
éventuellement, on prépare à partir des acides les sels, les complexes avec des métaux ou les sels complexes de métaux.

9. Composés selon les revendications 1 et 2, caractérisés en ce qu'au moins l'un des substituants X représente un ion de métal alcalin.

10. Composés selon les revendications 1, 2 et 9, caractérisés en ce qu'au moins une partie des substituants X représente les équivalents d'ions de métaux dont les numéros atomiques valent de 20 à 32, 42 à 44, 49 ou 57 a 83.

11. Complexes de gadolinium de l'acide $N^6$-carboxyméthyl-$N^3$,$N^9$-bis(trihydroxy-2,3,4 butyl-carbamoylméthyl)-triaza-3,6,9 undécanedioïque, de l'acide $N^6$-carboxyméthyl-$N^3$,$N^9$-bis(dihydroxy-2,3 propyl-carbamoylméthyl)-triaza-3,6,9 undécanedioïque, de l'acide $N^6$-carboxyméthyl-$N^3$,$N^9$-bis(dihydroxy-2,3 propyl-N-méthyl-carbamoylméthyl)-triaza-3,6,9 undécanedioïque, de l'acide $N^6$-carboxyméthyl-$N^3$,$N^9$-bis [hydroxy-2 (hydroxyméthyl)-1 éthyl-carbamoylméthyl]-triaza-3,6,9 undécanedioïque, de l'acide $N^6$-carboxyméthyl-$N^3$,$N^9$-bis[bis-(hydroxy-2 éthyl)-carbamoylméthyl]-triaza-3,6,9 undécanedioïque, de l'acide N-carboxyméthyl-$N^3$,-$N^9$-bis(pentahydroxy-2,3,4,5,6 hexyl-N--méthyl--carbamoylméthyl)-triaza-3,6,9 undécanedioïque et de l'acide $N^6$-carboxyméthyl-$N^3$,$N^9$-bis[dihydroxy--2,3 propyl(hydroxyméthyl)-1 carbamoylméthyl]--triaza-3,6,9 undécanedioïque.

12. Complexe de lanthane, de dysprosium, d'holmium, de terbium, d'ytterbium, de cérium, de samarium, de bismuth, de chrome et de fer de l'acide $N^6$--carboxyméthyl-$N^3$,$N^9$-bis(dihydroxy-2,3 propyl-N-méthyl-carbamoylméthyl)-triaza-3,6,9 undécanedioïque.

13. Complexes de manganèse du trans-diamino-1,2 N,N'-bis(carboxyméthyl)-N,N'-bis(dihydroxy-2,3 propyl-carbamoylméthyl)-cyclohexane, du trans-diamino-1,2 N,N'-bis(carboxyméthyl)-N,N'-bis(dihydroxy-2,3 propyl-N-méthyl-carbamoylméthyl)-cyclohexane, du trans-diamino-1,2 N,N'-bis-(carboxyméthyl)-N,N'-bis[bis(hydroxy-2-éthyl)-carbamoylméthyl]-cyclohexane, du trans-diamino-1,2 N,N'-bis(carboxyméthyl)-N,N'-bis(hydroxy-2 éthyl-carbamoylméthyl)-cyclohexane, du trans-diamino-1,2 N,N'-bis(carboxyméthyl)-N,N'-bis[hydroxy-2 (hydroxyméthyl)-1 éthyl-carbamoylméthyl]-cyclohexane et de l'acide $N^3$,$N^6$-bis(dihydroxy-2,3 propyl-N-méthyl-carbamoylméthyl)diaza-3,6 octanedioïque.

14. Sel de sodium du complexe de calcium de l'acide $N^6$-carboxyméthyl-$N^3$,$N^9$-bis(dihydroxy-2,3 propyl-carbamoylméthyl)-triaza-3,6,9 undécanedioïque.

15. Complexes de l'acide $N^6$-carboxyméthyl-$N^3$,$N^9$-bis(dihydroxy-2,3 propyl-carbamoylméthyl)-triaza-3,6,9 undécanedioïque avec $^{111}In$, $^{113m}In$, $^{67}Cu$, $^{67}Ga$, $^{169}Yb$, $^{153}Gd$ et $^{99m}Tc$.

16. Sel de mono-N-méthylglucamine du complexe de zinc de l'acide $N^6$-carboxyméthyl-$N^3$,$N^9$-bis(dihydroxy-2,3 propyl-N-méthyl-carbamoylméthyl)-triaza-3,6,9 undécanedioïque.

17. Produit pour diagnostic, contenant un composé selon les revendications 11 à 13 et 15.

18. Préparations pharmaceutique, contenant un composé selon les revendications 14 et 16.

19. Utilisation d'au moins un sel de complexe, physiologiquement compatible, de formule générale I

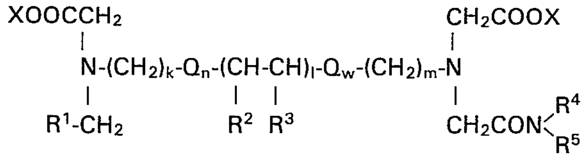

(I),

dans laquelle

$R^1$ représente un groupe COOX ou le groupe

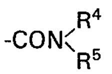

$R^2$ et $R^3$ sont identiques ou différents et représentent chacun un atome d'hydrogène, un reste d'hydrocarbure inférieur linéaire au ramifié, un groupe phényle ou benzyle ou bien $R^2$ ou $R^3$ représente le groupe

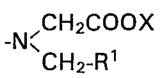

ou $R^2$ et $R^3$, pris ensemble, forment un reste triméthylène ou tétraméthylène,

$R^4$ représente un reste mono- ou poly-hydroxylé, liniaire ou ramifié,

$R^5$ représente un atome d'hydrogène, un reste d'hydrocarbure inférieur linéaire ou ramifié, éventuellement mono- ou poly-hydroxylé,

Q représente un atome d'oxygène ou de soufre ou le groupe $-N-(CH_2)_jR^x$, où j représente les chiffres 1 à 4 et $R^x$ représente un atome d'hydrogène, un groupe alcoxy inférieur ayant 1 à 4 atomes de carbone, un groupe COOX ou, quand j est supérieur à 1, le groupe

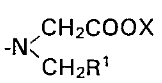

k et m sont identiques ou différents et représentent les chiffres 0 ou 2,

l représente les chiffres 1 ou 2 et n et w sont identiques ou différents et représentent les nombres 0 ou 1, n et w ne valant 1 que lorsque k et m valent 2, et les X représentent des atomes d'hydrogène et/ou des équivalents d'ions de métaux,

ainsi que leurs sels avec des bases organiques physiologiquement compatibles,

caractérisée en ce qu'au moins une partie des substituants X représente des equivalents d'ions de métaux dont les numéros atomiques vont de 20 à 32, 42 à 44, 49 ou 57 à 83, pour préparer des produits pour diagnostic.

20. Utilisation des complexes de gadolinium de l'acide $N^6$-carboxyméthyl-$N^3$,$N^9$-bis(trihydroxy-2,3,4 butyl-carbamoylméthyl)-triaza-3,6,9 undécanedioïque, de l'acide $N^6$-carboxyméthyl-$N^3$,$N^9$-bis-(dihydroxy-2,3 propyl-carbamoylméthyl)-triaza-3,6,9 undécanedioïque, de l'acide $N^6$-carboxyméthyl-$N^3$,$N^9$-bis(dihydroxy-2,3 propyl-N-méthyl-carbamoylméthyl)-triaza-3,6,9 undécanedioïque, de l'acide $N^6$-carboxyméthyl-$N^3$,$N^9$-bis[hydroxy-2 (hydroxyméthyl)-1 éthyl-carbamoylméthyl]-triaza-3,6,9 undécanedioïque, de l'acide $N^6$-carboxyméthyl-$N^3$,$N^9$-bis[bis(hydroxy-2 éthyl)-carbamoylméthyl]-triaza-3,6,9 undécanedioïque, de l'acide $N^6$-carboxyméthyl-$N^3$,$N^9$-bis(pentahydroxy-2,3,4,5,6 hexyl-N-méthyl-carbamoylméthyl)-triaza-3,6,9 undécanedioïque et de l'acide $N^6$-carboxyméthyl-$N^3$,-$N^9$-bis[dihydroxy-2,3 propyl(hydroxyméthyl)-1 carbamoylméthyl]-triaza-3,6,9 undécanedioïque pour préparer des produits pour diagnostic.

21. Utilisation du complexe de lanthane, de dysprosium, d'holmium, de terbium, d'ytterbium, de cérium, de samarium, de bismuth, de chrome et de fer de l'acide $N^6$-carboxyméthyl-$N^3$,$N^9$-bis(dihydroxy-2,3 propyl-N-méthyl-carbamoylméthyl)-triaza-3,6,9 undécanedioïque pour préparer des produits pour diagnostic.

22. Utilisation des complexes de manganèse du trans-diamino-1,2 N,N'-bis(carboxyméthyl)-N,N'-bis(dihydroxy-2,3 propyl-carbamoylméthyl)-cyclohexane, du trans-diamino-1,2 N,N'-bis(carboxyméthyl)-N,N'-bis(dihydroxy-2,3 propyl-N-méthyl-carbamoylméthyl)-cyclohexane, du trans-diamino-1,2 N,N'-bis(carboxyméthyl)-N,N'-bis[bis(hydroxy-2 éthyl)-carbamoylméthyl]-cyclohexane,du trans-diamino-1,2 N,N'-bis(carboxyméthyl)-N,N'-bis(hydroxy-2 éthyl-carbamoylméthyl)-cyclohexane, du trans-diamino-1,2 N,N'-bis(carboxyméthyl)-N,N'-

-bis[hydroxy-2 (hydroxyméthyl)-1 éthyl-carbamoyl-méthyl]-cyclohexane et de l'acide $N^3$,$N^6$-bis(dihydroxy-2,3 propyl-N-méthyl-carbamoylméthyl)-diaza-3,6 octanedioïque pour préparer des produits pour diagnostic.

23. Utilisation du sel de sodium du complexe de calcium de l'acide $N^6$-carboxyméthyl-$N^3$,$N^9$-bis(dihydroxy-2,3 propyl-carbamoylméthyl)-triaza-3,6,9 undécanedioïque pour préparer des produits pour diagnostic.

24. Utilisation des complexes de l'acide $N^6$-carboxyméthyl-$N^3$,$N^9$-bis(dihydroxy-2,3 propyl-carbamoylméthyl)-triaza-3,6,9 undécanedioïque avec $^{111}In$, $^{113m}In$, $^{67}Cu$, $^{67}Ga$, $^{169}Yb$, $^{153}Gd$ et $^{99m}Tc$, pour préparer des produits pour diagnostic.

25. Utilisation du sel de mono-N-méthylglucamine du complexe de zinc de l'acide $N^6$-carboxyméthyl-$N^3$,$N^9$-bis(dihydroxy-2,3 propyl-N-méthyl-carbamoylméthyl)-triaza-3,6,9 undécanedioïque pour préparer des produits pour diagnostic.